(19) Europäisches Patentamt
European Patent Office
Office européen des brevets

(11) **EP 4 707 331 A1**

(12) **EUROPEAN PATENT APPLICATION**
published in accordance with Art. 153(4) EPC

(43) Date of publication:
**11.03.2026 Bulletin 2026/11**

(21) Application number: 24875627.2

(22) Date of filing: **19.11.2024**

(51) International Patent Classification (IPC):
$C08K\ 5/00^{(2006.01)}$    $C08K\ 5/13^{(2006.01)}$
$C08K\ 5/37^{(2006.01)}$    $C08K\ 5/49^{(2006.01)}$
$C08K\ 5/098^{(2006.01)}$    $C08K\ 5/17^{(2006.01)}$
$C08L\ 67/02^{(2006.01)}$    $C08G\ 63/183^{(2006.01)}$
$C08J\ 11/10^{(2006.01)}$    $C07C\ 69/82^{(2006.01)}$

(52) Cooperative Patent Classification (CPC):
C07C 69/82; C08G 63/183; C08J 11/10;
C08K 5/00; C08K 5/098; C08K 5/13; C08K 5/17;
C08K 5/37; C08K 5/49; C08L 67/02

(86) International application number:
**PCT/KR2024/018265**

(87) International publication number:
**WO 2026/014623 (15.01.2026 Gazette 2026/03)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **12.07.2024 KR 20240092589**

(71) Applicant: **SK Chemicals Co., Ltd.**
**Seongnam-si, Gyeonggi-do 13494 (KR)**

(72) Inventors:
• **YOO, Young-Man**
**Seongnam-si, Gyeonggi-do 13494 (KR)**
• **HAN, Ye-Ji**
**Seongnam-si, Gyeonggi-do 13494 (KR)**

(74) Representative: **Dehns**
**10 Old Bailey**
**London EC4M 7NG (GB)**

(54) **RECYCLED POLYESTER RESIN COMPOSITION AND MOLDED ARTICLE MANUFACTURED THEREFROM**

(57)    The present invention relates to a recycled polyester resin composition comprising recycled dimethyl terephthalate prepared from waste polyester and to an article molded therefrom. Specifically, according to an embodiment of the present invention, as the recycled polyester resin composition comprises a recycled polyester resin comprising a repeat unit derived from a glycol component and a repeat unit derived from recycled dimethyl terephthalate (rDMT); a hindered phenol-based primary antioxidant; and a phosphorus-based or sulfur-based secondary antioxidant, it is possible to enhance crystallinity, thermal resistance, and volatility resistance. Accordingly, when a molded article for a vehicle headlamp is prepared using the recycled polyester resin composition, excellent performance can be exhibited.

[Fig. 1]

**Description**

**Technical Field**

The present invention relates to a recycled polyester resin composition comprising recycled dimethyl terephthalate prepared from waste polyester and to an article molded therefrom.

**Background Art**

Polyester is widely used as a material for beverage-filling containers, packaging films, audio and video films, and the like, or an industrial material such as medical fibers and tire cords, by virtue of its excellent mechanical strength, thermal resistance, transparency, and gas barrier properties. In particular, polyester sheets or plates have good transparency and excellent mechanical strength, so that they are widely used as raw materials for cases, boxes, partitions, shelves, panels, packaging materials, building materials, interior and exterior materials, and the like.

As a result, waste of plastics such as polyester is generated globally at an unmanageable level every year. Recently, countries around the world have prepared regulations and plans for recycling waste plastic resources, including waste polyester. Physical or chemical methods are used as methods of recycling waste polyester. Physical recycling methods cannot guarantee purity and, therefore, are not widely used. In chemical recycling methods, the ester bond of waste polyester is cleaved to depolymerize it. Reactions such as glycolysis, hydrolysis, methanolysis, and aminolysis may be used.

In recent years, waste polyester is not simply recycled; rather, research is ongoing to enhance the quality of polyester products manufactured using recycled raw materials prepared by recycling waste polyester. For example, there have been attempts to recycle waste polyethylene terephthalate (PET) through glycolysis to prepare recycled polyester resins other than PET. However, since an excessive amount of a glycol is used in this reaction and the reaction time is long, there is a problem in that a large amount of by-products are formed by a side reaction. In addition, when PET is recycled through methanolysis to prepare recycled polyester resins other than PET, there is a problem in that the properties of the final resin prepared and the products produced using the same are significantly deteriorated, resulting in lower quality.

Meanwhile, examples of polyester products include materials for vehicle headlamps or headlights. A vehicle headlamp comprises a reflector to focus light from a light source and direct it forward and a bezel to accommodate the reflector. The bezel and reflector are manufactured by depositing metal on the surface of a substrate and require a complex design and high gloss. High fluidity of a material is required to manufacture a molded article with such a complex design. That is, it is possible to manufacture a molded article with high quality without any unmolded portion even within a complex mold structure by using a material with high fluidity. In recent years, polybutylene terephthalate (PBT) resins have been used as a material that satisfies the high fluidity and high smoothness required for molded articles such as vehicle headlamps as they have a fast crystallization speed, a short molding period, and excellent fluidity, making it easy to implement complex shapes. However, as heat sources diversify due to the increase in light sources and electrical components in functional aspects of headlamp bezels and reflectors, designs are also becoming increasingly complex. These various heat sources and the complex structure of the headlamp may cause a temperature difference between the inside and outside of the headlamp, and the headlamp component material may be thermally decomposed, which increases the possibility of the component becoming hazy. Accordingly, the level of physical properties required for resins used as materials is gradually increasing.

Therefore, research is ongoing on recycled polyester resins and products produced using the same, in which the properties of the final resins prepared using the same and the products produced therefrom are not deteriorated, resulting in excellent quality, while the formation of by-products is minimized since an excessive amount of alcohol or glycol is not used in the process of recycling waste polyester.

For example, Korean Laid-open Patent Publication No. 2011-0080260 discloses a method for chemically recycling a polyester, which is readily used to produce fine fibers, functional yarns, and the like, by depolymerizing waste polyester and ethylene glycol to prepare an oligomer solution, bring the oligomer solution into contact with an ion-exchange resin to remove a catalyst in the oligomer solution, passing the oligomer solution from which the catalyst has been removed through a decolorizing agent to remove colored substances such as a colorant in the oligomer solution, and subjecting the solution to polycondensation.

[Prior Art Document]

(Patent Document 1) Korean Laid-open Patent Publication No. 2011-0080260

**Disclosure of Invention**

## Technical Problem

Accordingly, the present invention aims to provide a recycled polyester resin composition, which can minimize the formation of by-products in the process of depolymerizing waste polyester and can produce high-purity recycled dimethyl terephthalate with excellent processability, along with excellent physical properties, by virtue of comprising the recycled dimethyl terephthalate, and an article molded therefrom.

## Solution to Problem

The recycled polyester resin composition according to an embodiment of the present invention comprises a recycled polyester resin comprising a repeat unit derived from a glycol component and a repeat unit derived from recycled dimethyl terephthalate (rDMT); a hindered phenol-based primary antioxidant; and a phosphorus-based or sulfur-based secondary antioxidant, wherein in the recycled polyester resin, the crystallized area value is 20% or more when calculated according to the following Equation 1 for the crystallized region and the non-crystallized region obtained by separating the crystal diffraction peaks appearing at 2θ values of 0° to 50° by the individual peak fitting method in an X-ray diffraction (XRD) analysis.

$$\text{Crystallized area value (\%)} = (\text{area of crystallized region})/(\text{area of crystallized region} + \text{area of non-crystallized region}) \times 100 \qquad \text{[Equation 1]}$$

The molded article according to another embodiment of the present invention is prepared from the recycled polyester resin composition.

## Advantageous Effects of Invention

As the recycled polyester resin composition according to an embodiment of the present invention comprises a recycled polyester resin comprising a repeat unit derived from a glycol component and a repeat unit derived from recycled dimethyl terephthalate (rDMT); a hindered phenol-based primary antioxidant; and a phosphorus-based or sulfur-based secondary antioxidant, it is possible to enhance crystallinity, thermal resistance, and volatility resistance.

In general, recycled dimethyl terephthalate (rDMT) prepared from waste polyester has low purity due to impurities resulting from side reactions with reagents used in the depolymerization process. Therefore, attempts have been made to enhance purity through processes such as fractional distillation; however, there are problems with low efficiency and difficulty in commercial use in terms of process costs, and there are also limits to enhancing purity.

As the recycled polyester resin composition according to an embodiment of the present invention comprises recycled dimethyl terephthalate prepared by depolymerizing waste polyester and having excellent color, purity, and crystallinity, it is excellent in crystallinity, thermal resistance, and volatility resistance.

Specifically, the recycled dimethyl terephthalate is prepared by crystallizing a depolymerization composition prepared by depolymerizing waste polyester at a low temperature, subjecting the slurry produced through the crystallization to solid-liquid separation, and drying, washing, and then purifying the product, whereby it is excellent in color, purity, and crystallinity. More specifically, in the process for preparing recycled dimethyl terephthalate, the content of ethylene glycol-based by-products that may be formed by side reactions in the process or terephthalate-based derivatives such as methyl hydrogen terephthalate (MHT), other than the desired DMT, is low. Thus, it is possible to enhance color characteristics and purity and to enhance quality when used as a raw material for various polymers. In addition, since recycled dimethyl terephthalate with high purity can be prepared in the process for preparing recycled dimethyl terephthalate, its processability is excellent. Further, it is environmentally friendly since the content of ethylene glycol-based by-products that can have an environmental impact is low.

Therefore, since the recycled polyester resin is prepared using rDMT with excellent purity, crystallinity, and color characteristics, it is excellent in such properties as color such as color-b and color-L, melting point (Tm), melting enthalpy, and heat deflection temperature, as well as crystallinity. As a result, products composed solely of the recycled polyester resin without mixing with commercially available polyester resins also have excellent quality.

Further, the recycled polyester resin having excellent properties as described above is used together with a hindered phenol-based primary antioxidant and a phosphorus-based or sulfur-based secondary antioxidant, while the contents thereof are controlled. As a result, when a molded article for a vehicle headlamp, more specifically a bezel or a reflector, is manufactured using the recycled polyester resin composition having such features, excellent performance can be exhibited.

**Brief Description of Drawings**

Fig. 1 shows X-ray diffraction (XRD) analyses of resins A, D, E, H, and I.
Figs. 2 to 6 each show peaks separated by deconvolution of X-ray diffraction peaks of resins A, D, E, H, and I using the individual peak fitting method.

**Best Mode for Carrying out the Invention**

Hereinafter, the present invention will be described in detail. The present invention is not limited to the disclosures given below, but it may be modified into various forms as long as the gist of the invention is not changed.

Throughout the present specification, when a part is referred to as "comprising" an element, it is understood that other elements may be comprised, rather than other elements are excluded, unless specifically stated otherwise.

All numbers and expressions related to the quantities of components, reaction conditions, and the like used herein are to be understood as being modified by the term "about," unless otherwise indicated.

Throughout the present specification, the terms first, second, and the like are used to describe various components. But the components should not be limited by the terms. The terms are used only for the purpose of distinguishing one component from another.

In the present specification, a unit or group "derived" from a specific component refers to a part of the component contained in a final product through a chemical reaction such as a polymerization reaction. It may be present in a form in which a part thereof is modified during the reaction or it is combined with other components. For example, a unit or group derived from at least one monomer is contained in the chain constituting a polymer.

**Recycled polyester resin composition**

The recycled polyester resin composition according to an embodiment of the present invention comprises a recycled polyester resin comprising a repeat unit derived from a glycol component and a repeat unit derived from recycled dimethyl terephthalate (rDMT); a hindered phenol-based primary antioxidant; and a phosphorus-based or sulfur-based secondary antioxidant, wherein in the recycled polyester resin, the crystallized area value is 20% or more when calculated according to the above Equation 1 for the crystallized region and the non-crystallized region obtained by separating the crystal diffraction peaks appearing at 20 values of 0° to 50° by the individual peak fitting method in an X-ray diffraction (XRD) analysis.

The recycled polyester resin composition may have a melt flow index (MI) of 60 g/10 minutes or more as measured under the conditions of 250°C and 2.16 kg according to ASTM D1238. For example, the melt flow index (MI) of the recycled polyester resin composition as measured under the conditions of 250°C and 2.16 kg according to ASTM D1238 may be 60.5 g/10 minutes or more, 61 g/10 minutes or more, 62 g/10 minutes or more, 65 g/10 minutes or more, 70 g/10 minutes or more, 72 g/10 minutes or more, 76 g/10 minutes or more, 80 g/10 minutes or more, 85 g/10 minutes or more, or 90 g/10 minutes or more.

The recycled polyester resin composition may have a tensile strength of 45 MPa or more according to ASTM D638. For example, the tensile strength of the recycled polyester resin composition according to ASTM D638 may be 46 MPa or more, 48 MPa or more, 49 MPa or more, 50 MPa or more, 52 MPa or more, 55 MPa or more, 57 MPa or more, or 58 or more.

The recycled polyester resin composition may have a heat deflection temperature (HDT) of 175°C or higher as measured at a low load of 0.45 MPa according to ASTM D648. For example, the heat deflection temperature (HDT) of the recycled polyester resin composition as measured at a low load of 0.45 MPa according to ASTM D648 may be 176°C or higher, 178°C or higher, 180°C or higher, 181°C or higher, 182°C or higher, 183°C or higher, 185°C or higher, 186°C or higher, or 188°C or higher.

The recycled polyester resin composition may have a TVOC peak area of 80 area/g or less as measured by a gas chromatography/mass spectrometry (GC/MS) analysis method. For example, the TVOC peak area of the recycled polyester resin composition as measured by a gas chromatography/mass spectrometry (GC/MS) analysis method may be 65 area/g or less, 60 area/g or less, 55 area/g or less, 50 area/g or less, 48 area/g or less, 46 area/g or less, 44 area/g or less, 41 area/g or less, or 40 area/g or less.

In addition, the recycled polyester resin composition may have a change in haze of 5 or less according to the following Equation 2.

[Equation 2]

$$\text{Change in haze} = |H1 - H2|$$

In Equation 2, H1 is the haze measured before fogging evaluation under the conditions of 130°C and 5 hours according to the DIN 75201-A test method for pellets prepared using the recycled polyester resin composition, which is a numerical value excluding the unit, and H2 is the haze due to gas formed during fogging evaluation under the conditions of 130°C and 5 hours according to the DIN 75201-A test method for pellets prepared using the recycled polyester resin composition, which is a numerical value excluding the unit.

For example, the change in haze of the recycled polyester resin composition according to the above Equation 2 may be 4.5 or less, 4 or less, 3.5 or less, 3 or less, 2.9 or less, 2.8 or less, 2.7 or less, 2.5 or less, or 2.2 or less.

In addition, when pellets prepared using the recycled polyester resin composition are dried under a reduced pressure at 50°C for 15 hours and heated from 30°C to 240°C at 10°C/minute using a differential scanning calorimeter (DSC, TA Instruments) in a nitrogen atmosphere, and when the conditions are then changed from nitrogen to air at a conversion rate of 20 ml/minute, the oxidation exotherm time measured while 240°C is maintained for 60 minutes may be 10 minutes or more. For example, the oxidation exotherm time may be 10.5 minutes or more, 11 minutes or more, 12 minutes or more, 13 minutes or more, 15 minutes or more, 17 minutes or more, 17.5 minutes or more, 20 minutes or more, 25 minutes or more, 35 minutes or more, 50 minutes or more, or 60 minutes or more.

In addition, the recycled polyester resin composition may have a melting point of 210°C or higher and a melting enthalpy ($\Delta$Hm) of 38 J/g or more as measured in a second scan using a differential scanning calorimeter. For example, the melting point may be 215°C or higher, 218°C or higher, 220°C or higher, 222°C or higher, or 225°C or higher, and 300°C or lower, 280°C or lower, 260°C, or 240°C or lower. The melting enthalpy ($\Delta$Hm) may be 39.5 J/g or more, 40 J/g or more, 40.5 J/g or more, 41 J/g or more, 41.3 J/g or more, 42 J/g or more, 42.5 J/g or more, 43 J/g or more, 43.2 J/g or more, or 43.5 J/g or more.

Recycled polyester resin

The recycled polyester resin composition according to an embodiment of the present invention comprises a recycled polyester resin comprising a repeat unit derived from a glycol component and a repeat unit derived from recycled dimethyl terephthalate (rDMT).

The recycled polyester resin may comprise a repeat unit derived from one or more glycol components; and a repeat unit derived from one or more acid components.

The recycled polyester resin may be a homopolymer or copolymer resin. As an example, the recycled polyester resin may be a polybutylene terephthalate (PBT) resin, which is a homopolymer resin comprising 1,4-butanediol as a constituent glycol component and recycled dimethyl terephthalate as a constituent acid component. As another example, the recycled polyester resin may be a copolymer resin comprising 1,4-butanediol and an additional glycol as a constituent glycol component, and recycled dimethyl terephthalate and an additional acid as a constituent acid component. The additional glycol and acid components may be additionally added in the esterification reaction step and/or in the polycondensation reaction step, which will be described in detail in the process for preparing a recycled polyester resin below.

According to an embodiment of the present invention, the recycled polyester resin comprises a repeat unit derived from a glycol component.

The glycol component may comprise at least one selected from the group consisting of 1,3-propanediol, 1,4-cyclohexanedimethanol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 2,3-butanediol, 3-methyl-1,5-pentanediol, 3-methyl-2,4-pentanediol, 1,6-hexanediol, 1,2-cyclohexanediol, 1,4-cyclohexanediol, diethylene glycol, polyethylene glycol, polypropylene glycol, polytetramethylene glycol, polyhexamethylene glycol, copolymers of ethylene oxide and tetrahydrofuran, ethylene oxide-adducted polypropylene glycol, polycarbonate diol, polyneopentyl glycol, poly-3-methylpentanediol, and poly-1,5-pentanediol.

The recycled dimethyl terephthalate may be prepared by depolymerizing waste polyester. Alternatively, the recycled dimethyl terephthalate may be prepared by first depolymerizing waste polyester to obtain recycled bis(2-hydroxyethyl) terephthalate (rBHET) and second depolymerizing the same. The process for preparing recycled dimethyl terephthalate will be described in detail below.

Conventionally, a recycled terephthalate prepared from waste polyester has poor physical properties such as color characteristics and low purity, making it difficult to be used alone as an acid component as a raw material for a polyester resin. However, a recycled polyester resin according to an embodiment of the present invention is prepared using the recycled terephthalate; thus, it has excellent quality since sufficient physical properties can be secured even when the content of the recycled terephthalate is increased relative to the conventional one. Therefore, the recycled polyester resin according to an embodiment of the present invention may comprise the recycled terephthalate alone or may comprise it together with another acid component to enhance physical properties and the like.

The acid component may comprise at least one selected from the group consisting of adipic acid, sebacic acid, succinic acid, isodecylsuccinic acid, maleic acid, fumaric acid, glutaric acid, azelaic acid, 1,4-cyclohexanedicarboxylic acid, 1,3-cyclohexanedicarboxylic acid, terephthalic acid, isophthalic acid, naphthalenedicarboxylic acid, diphenyldicarboxylic acid, 4,4'-stilbenedicarboxylic acid, 2,5-furandicarboxylic acid, and 2,5-thiophenedicarboxylic acid.

The recycled dimethyl terephthalate may have a purity of 90% or more. For example, the purity of the recycled dimethyl terephthalate may be obtained by measuring the area of a peak using ultra-performance liquid chromatography (UPLC) and then calculating its fraction (%) relative to the total peak area. It may be 91% or more, 93% or more, 95% or more, 97% or more, 98% or more, 99% or more, 99.5% or more, or 99.9% or more.

Specifically, 0.01 g of a sample is diluted in 20 ml of a mixture of methanol and 0.3% phosphoric acid solution in distilled water. The area of a peak is measured using ultra-performance liquid chromatography (UPLC), and its fraction (%) relative to the total peak area is then calculated to determine the purity, the components formed by side reactions, the components formed other than the target DMT, and their contents. In such an event, the dilution may be performed depending on the mobile phase and flow rate.

In addition, the recycled dimethyl terephthalate may have an APHA color value of 120 or less. For example, it may be measured by dissolving the recycled dimethyl terephthalate in a dimethylformamide solvent at a concentration of 10% by weight, placing it in an analysis vessel made of quartz with a light path length of 10 mm, and then performing measurement in the transmittance mode of a colorimeter, according to ASTM-D1209. It may be 105 or less, 100 or less, 93 or less, 90 or less, 85 or less, 65 or less, or 55 or less. APHA (American Public Health Association) is a color-coded scale that indicates the level of contamination in liquid. The lower the number, the closer it is to the color of pure water (colorless). As the recycled dimethyl terephthalate has an APHA color value satisfying the above range, it has high purity and low contamination by impurities, resulting in excellent quality.

According to an embodiment of the present invention, the crystallized area value is 20% or more when calculated according to the following Equation 1 for the crystallized region and the non-crystallized region obtained by separating the crystal diffraction peaks appearing at 2θ values of 0° to 50° by the individual peak fitting method in an X-ray diffraction (XRD) analysis of the recycled polyester resin.

Crystallized area value (%) = (area of crystallized region)/(area of crystallized region + area of non-crystallized region) × 100      [Equation 1]

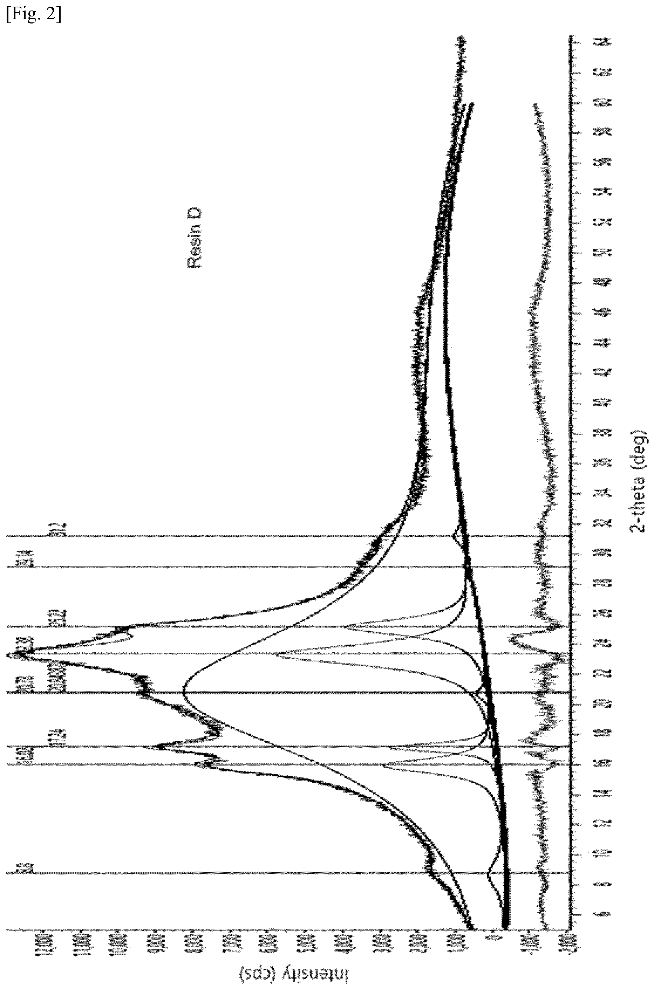

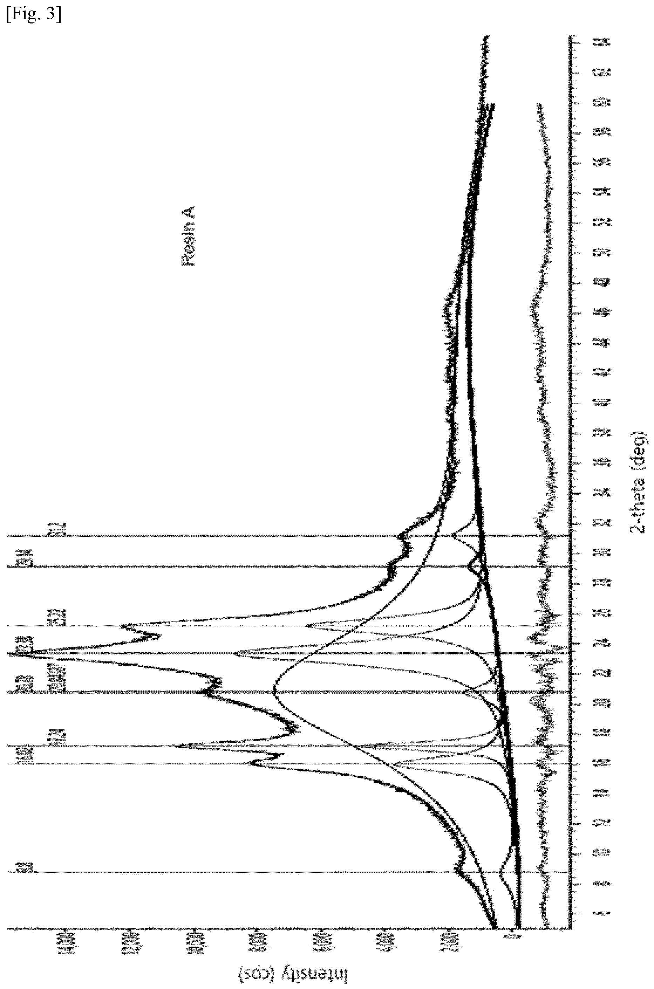

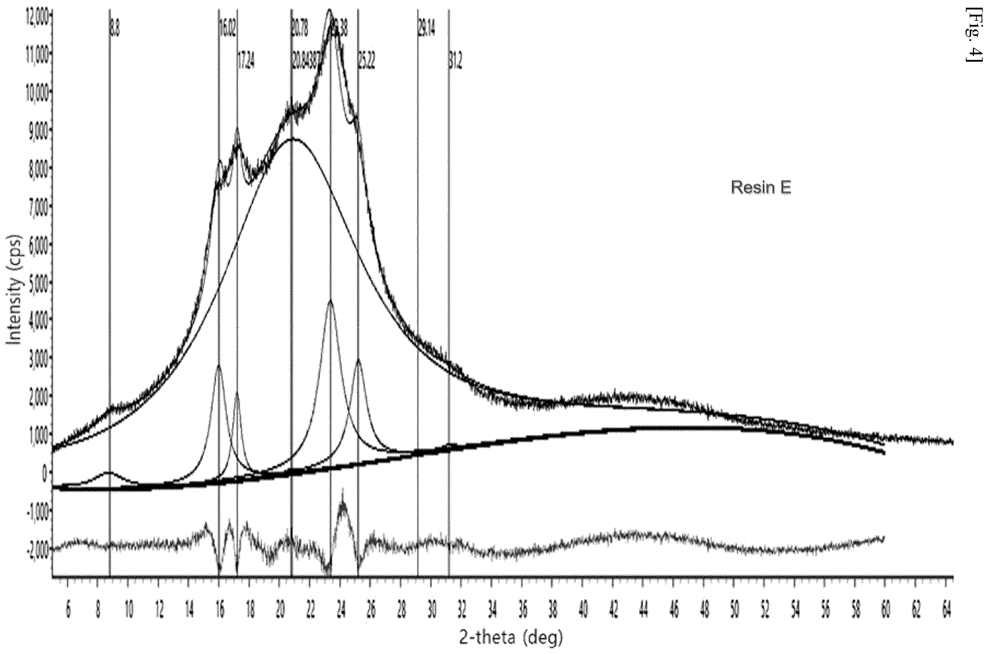

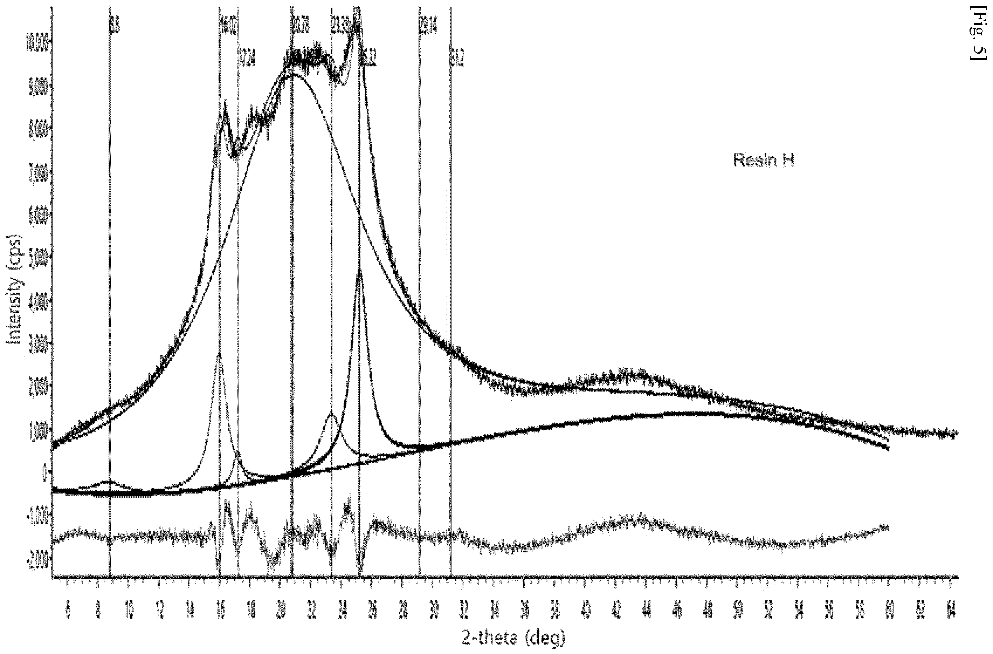

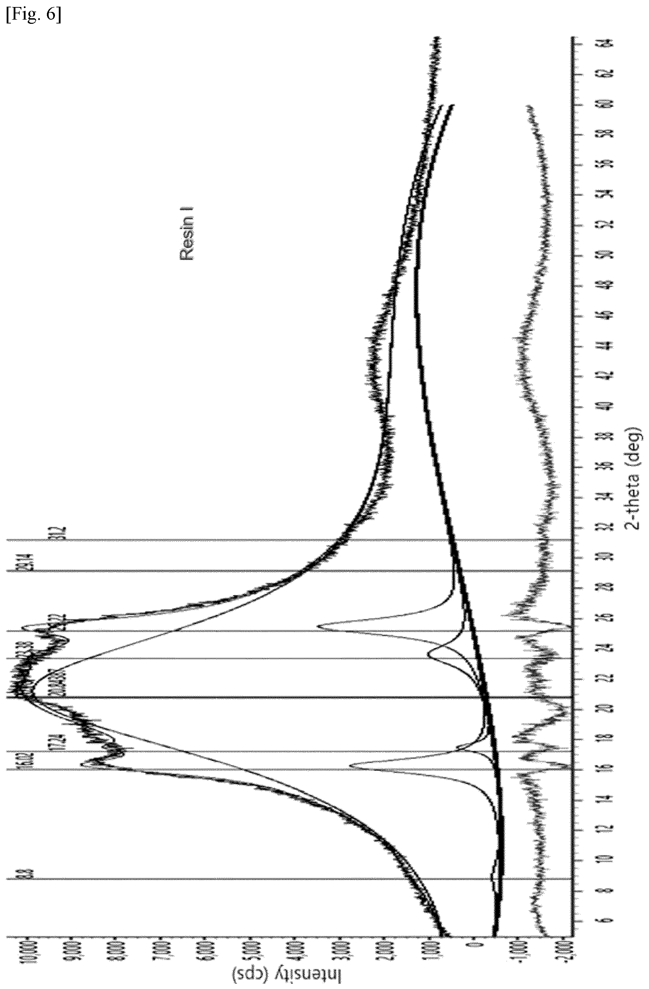

Here, the crystallized area value (%) refers to the percentage (%) of the area of the crystallized region relative to the total area measured in an X-ray diffraction (XRD) analysis of the polyester resin, i.e., the sum of the areas of the crystallized region and the non-crystallized region. The area of each region, which is the basis for calculating the crystallized area value (%) according to the present invention, is obtained by separating peaks measured in an X-ray diffraction (XRD) analysis of the polyester resin at 2θ values of 0° to 50° by the individual peak fitting method. It may be calculated as an integral value for the crystallized region or the non-crystallized region, respectively. Since the crystalline components are arranged regularly in three-dimensional space, scattering occurs in a certain direction when X-rays collide with the diffraction surface; thus, the diffraction peak in the crystallized region appears as a high and narrow peak. In contrast, since the amorphous components are arranged irregularly, scattering occurs in various directions; thus, the peak of the non-crystallized region appears gently over a wide 2θ range (see Figs. 2 to 6).

The larger the crystallized area value according to the above Equation 1, more specifically, the larger the ratio of each crystallized area separated by the individual peak fitting method, the better the crystallinity of the resin. In addition, such properties of the resin as mechanical strength, thermal resistance, and chemical resistance can also be enhanced. However, as the crystallinity increases, the adhesive strength decreases at temperatures lower than the melting point (Tm), which may limit the use of the resin; thus, it is important to control the properties to appropriate ranges.

For example, the crystallized area value of the recycled polyester resin according to the above Equation 1 may be 20.5% or more, 21% or more, 21.5% or more, 22% or more, 22.5% or more, 23% or more, 25% or more, 26% or more, 27% or more, 29% or more, 30% or more, 31% or more, 33% or more, or 33.5% or more. As the crystallized area value of the recycled polyester resin according to the above Equation 1 satisfies the above range, it has desirable ranges of properties such as mechanical properties, thermal resistance, chemical resistance, and adhesive strength, along with excellent crystallinity, whereby it can be applied to various purposes.

The XRD analysis according to the above Equation 1 may be a WAXD (wide angle X-ray diffraction) analysis and may be performed by taking a photograph of a WAXD plate using a SmartLab type X-ray diffraction device from Rigaku. In the analysis of the above WAXD, correction data that has been subjected to a polarization factor, an absorption factor, and an air scattering correction for the measured data can be used, and X-ray diffraction (XRD) analysis deconvolution and amorphous component region curve separation can be performed, and the integral value for the crystallized region diffraction peak at 20 values of 0° to 50° and the non-crystalline region scattering intensity is used to calculate the crystallized area value (%) according to the above Equation 1.

In addition, the recycled polyester resin may have a negative value of the difference in the crystallized area value according to the following Equation 3.

$$[\text{Equation 3}]$$

$$\text{Difference in crystallized area value} = Ca_1 - Ca_2$$

In Equation 3, $Ca_1$ is the crystallized area value calculated according to the above Equation 1 for a polyester resin comprising a repeat unit derived from dimethyl terephthalate (virgin DMT), which is a numerical value excluding the unit, and $Ca_2$ is the crystallized area value calculated according to the above Equation 1 for the recycled polyester resin comprising a repeat unit derived from recycled dimethyl terephthalate (rDMT), which is a numerical value excluding the unit.

Specifically, $Ca_1$ is the crystallized area value calculated according to the above Equation 1 for a polyester resin comprising a repeat unit derived from commercially available dimethyl terephthalate, rather than recycled dimethyl terephthalate prepared from waste polyester, which is a numerical value excluding the unit. For example, $Ca_1$ may be 20 or more, 20.5 or more, 21 or more, 21.5 or more, or 22 or more.

$Ca_2$ is the crystallized area value calculated according to the above Equation 1 for the recycled polyester resin comprising a repeat unit derived from recycled dimethyl terephthalate prepared from waste polyester, which is a numerical value excluding the unit. For example, $Ca_2$ may be 20.5 or more, 21 or more, 21.5 or more, 22 or more, 22.5 or more, 23 or more, 25 or more, 25.5 or more, 27 or more, 29 or more, 30 or more, 31.5 or more, 32 or more, or 33.5 or more, as described above.

The difference in the crystallized area value according to the above Equation 3 may be -0.5 or less, -1 or less, -2 or less, -3 or less, -4 or less, -5 or less, -6 or less, -7 or less, -8 or less, -9.5 or less, -10 or less, -11 or less, or -12 or less. As the difference in the crystallized area value according to the above Equation 3 satisfies the above range, it has superior crystallinity to polyester resins comprising commercially available dimethyl terephthalate rather than recycled dimethyl terephthalate.

In addition, the recycled polyester resin may have a total content (% by mole) of an ethylene glycol residue and a diethylene glycol residue of 5% by mole or less based on the total number of moles of the glycol component residues. Specifically, the recycled polyester resin may comprise an ethylene glycol residue. More specifically, it may comprise an ethylene glycol residue and a diethylene glycol residue. For example, the polyester resin may comprise an ethylene glycol residues and a diethylene glycol residue in an amount of 3.5% by mole or less, 2.5% by mole or less, 2% by mole or less, 1.8% by mole or less, or 1.5% by mole or less, and 0.01% by mole or more, 0.02% by mole or more, 0.03% by mole or more, 0.05% by mole or more, or 0.1% by mole or more, based on the total number of moles of the glycol component residues.

The recycled polyester resin may have an intrinsic viscosity of 0.5 dl/g to 1.5 dl/g at 35°C. For example, the intrinsic viscosity of the recycled polyester resin at 35°C may be 0.55 dl/g to 1.35 dl/g, 0.65 dl/g to 1.3 dl/g, 0.7 dl/g to 1.2 dl/g, 0.72 dl/g to 1.14 dl/g, 0.55 dl/g to 1.1 dl/g, or 0.6 dl/g to 0.7 dl/g.

The recycled polyester resin may have a color-b of 12 or less. For example, the color-b of the recycled polyester resin may be 10 or less, 9 or less, 8.5 or less, 8.1 or less, 7.5 or less, or 7 or less, and greater than 0, 1 or more, 2 or more, 2.5 or more, or 3 or more.

The recycled polyester resin may have a color-L of 70 or more. For example, the color-L of the recycled polyester resin may be 72 or more, 75 or more, 78 or more, 80 or more, 81 or more, 82 or more, or 83 or more.

The color characteristics are a color coordinate established by the Commission International d'Eclairage (CIE), where color is represented by L (brightness), a (green to red complementary color), and b (yellow to blue complementary color). It can be measured using a colorimeter.

In addition, the recycled polyester resin may have a melting point of 210°C or higher and a melting enthalpy ($\Delta Hm$) of 38 J/g or more as measured in a second scan using a differential scanning calorimeter. For example, the melting point may be 215°C or higher, 218°C or higher, 220°C or higher, 222°C or higher, or 225°C or higher, and 300°C or lower, 280°C or lower, 260°C, or 240°C or lower. The melting enthalpy ($\Delta Hm$) may be 39.5 J/g or more, 40 J/g or more, 40.5 J/g or more, 41 J/g or more, 41.3 J/g or more, 42 J/g or more, 42.5 J/g or more, 43 J/g or more, 43.2 J/g or more, or 43.5 J/g or more.

Specifically, the melting enthalpy may be measured by drying the recycled polyester resin under a reduced pressure at 50°C for 15 hours, melting it at 260°C, then rapidly cooling it to 30°C, and using a differential scanning calorimeter to scan it at a temperature elevation rate of 10°C/minute. In such an event, melting enthalpy may be measured by using a differential scanning calorimeter by a first scan (1st scan) or a second scan (2nd scan). In the present specification, it is measured by a second scan.

In the heat flow curve obtained by scanning, the first endothermic temperature is a glass transition temperature (Tg), the exothermic temperature measured after the glass transition temperature (Tg) is a crystallization temperature (Tc), and the endothermic temperature measured after the crystallization temperature (Tc) is a melting point (Tm). In addition, the integral value at the melting point (Tm) is calculated as melting enthalpy ($\Delta$Hm).

In addition, the recycled polyester resin may have a heat deflection temperature (HDT) of 90°C or higher as measured at a low load of 0.48 MPa according to ASTM D648. For example, the heat deflection temperature may be 92°C or higher, 94.5°C or higher, 95°C or higher, 96°C or higher, 98°C or higher, 98.4°C or higher, 99°C or higher, 99.3°C or higher, 99.5°C or higher, 100°C or higher, or 101°C or higher.

Primary antioxidant

The recycled polyester resin composition according to an embodiment of the present invention comprises a primary antioxidant.

The primary antioxidant may be a phenol-based antioxidant. The phenol-based antioxidant can prevent oxidation of the recycled polyester resin composition while also playing a role in thermally stabilizing the recycled polyester resin composition. The type of phenol-based antioxidant is not particularly limited, and various commercially available phenol-based antioxidants may be used. As a specific example, the recycled polyester resin composition according to an embodiment of the present invention comprises a hindered phenol-based primary antioxidant.

For example, the primary antioxidant may comprise tetrakis[methylene-3-(3,5-di-tert-butyl-4-hydroxyphenyl) propionate]methane, octadecyl-3-(3,5-di-tert-butyl-4-hydroxyphenyl)propionate, benzenepropanoic acid, 3,5-bis(1,1-dimethylethyl)-4-hydroxy, alkyl ester (wherein the alkyl has 7 or 9 carbon atoms), triethylene glycol-bis-3-(3-tert-butyl-4-hydroxy-5-methylphenyl)propionate, tris(3,5-di-tert-butyl-4-hydroxybenzyl)isocyanurate, 1,3,5-trimethyl-2,4,6-tris(3,5-di-tert-butyl-4-hydroxybenzyl)benzene, or a combination thereof.

The recycled polyester resin composition may comprise the primary antioxidant in an amount of 0.001 part by weight to 0.3 part by weight relative to 100 parts by weight of the recycled polyester resin. For example, the content of the primary antioxidant may be 0.002 part by weight to 0.3 part by weight, 0.005 part by weight to 0.3 part by weight, 0.01 part by weight to 0.3 part by weight, 0.01 part by weight to 0.25 part by weight, 0.02 part by weight to 0.2 part by weight, or 0.05 part by weight to 0.1 part by weight, relative to 100 parts by weight of the recycled polyester resin.

As the content of the primary antioxidant satisfies the above range, the recycled polyester resin composition and an article molded therefrom can be advantageous in minimizing discoloration or gas generation due to the decomposition of the recycled polyester resin composition by heat at high temperatures, thereby securing an excellent appearance. If the content is less than the above range, an oxidation reaction is likely to take place in a high-temperature environment, which may result in the deterioration of physical properties. If the content exceeds the above range, decomposition products of the antioxidant may be migrated to the surface for a long period of time in a high-temperature environment, which may result in poor fogging evaluation.

The effect of the antioxidant on the thermal stability of a polyester resin at high temperatures may be confirmed by the oxidation induction time (OIT). Many organic compounds, including polymers, tend to undergo oxidation reactions even at low temperatures in the presence of oxygen. However, some substances have an induction period during which no reaction takes place with oxygen when exposed to isothermal conditions. This period is called the oxidation induction time (OIT). After this induction period, the rate of reaction with oxygen increases. That is, OIT stands for oxidation stability information of a substance. When the temperature is rapidly increased to a desired temperature and stabilized in a nitrogen atmosphere using DSC (differential scanning calorimetry), then switched to an oxygen atmosphere, and maintained in an isothermal state, the time at which an exothermic reaction is initiated is designated as OIT.

Secondary antioxidant

The recycled polyester resin composition according to an embodiment of the present invention comprises a secondary antioxidant. Specifically, the recycled polyester resin composition according to an embodiment of the present invention comprises a phosphorus-based or sulfur-based secondary antioxidant.

For example, the phosphorus-based antioxidant may comprise 3,9-bis(2,4-dicumylphenoxy)-2,4,8,10-tetraoxa-3,9-diphosphaspiro[5.5]undecane, bis(2,6-di-tert-butyl-4-methylphenyl)pentaerythritol-di-phosphite, bis(2,4-di-tert-butylphenyl)pentaerythritol-di-phosphite, tetrakis(2,4-di-tert-butylphenyl)4,4'-biphenylene diphosphonite, or a combination thereof.

In addition, for example, the sulfur-based antioxidant may comprise dilauryl-3,3'-thiodipropionic acid ester, dimyristyl-3,3'-thiodipropionic acid ester, distearyl-3,3'-thiodipropionic acid ester, laurylstearyl-3,3'-thiodipropionic acid ester, pentaerythrityl tetrakis(3-laurylthiopropion ester), or a combination thereof.

The recycled polyester resin composition may comprise the secondary antioxidant in an amount of 0.001 part by weight to 0.2 part by weight relative to 100 parts by weight of the recycled polyester resin. For example, the content of the secondary antioxidant may be 0.002 part by weight to 0.2 part by weight, 0.005 part by weight to 0.2 part by weight, 0.01 part by weight to 0.2 part by weight, or 0.02 part by weight to 0.18 part by weight, relative to 100 parts by weight of the recycled polyester resin.

As the content of the secondary antioxidant satisfies the above range, the recycled polyester resin composition and an article molded therefrom can be advantageous in minimizing discoloration or gas generation due to the decomposition of the recycled polyester resin composition by heat at high temperatures, thereby securing an excellent appearance. If the content is less than the above range, an oxidation reaction is likely to take place in a high-temperature environment, which may result in the deterioration of physical properties. If the content exceeds the above range, decomposition products of the antioxidant may be migrated to the surface for a long period of time in a high-temperature environment, which may result in poor fogging evaluation.

Wax-based slip agent

The recycled polyester resin composition according to an embodiment of the present invention may comprise a wax-based slip agent.

As a wax-based slip agent is employed, it can be advantageous in securing releasability during injection molding of an article prepared using the recycled polyester resin composition.

The wax may comprise at least one of a fatty acid ester-based lubricant and a montan-based lubricant.

The fatty acid ester-based lubricant may comprise at least one selected from the group consisting of fatty acid esters of alcohols or polyhydric alcohols, hardened oils, butyl stearate, stearic acid monoglyceride, pentaerythritol tetrastearate, stearyl stearate, ester wax, and alkyl phosphate esters.

The montan-based lubricant may comprise at least one of a montan acid ester wax and a montan acid metal salt. The montan acid ester wax may have a saponification value of 20 mg KOH/g to 300 mg KOH/g or 50 mg KOH/g to 250 mg KOH/g. When the montan acid ester wax having a saponification value within the above range is employed, the recycled polyester resin composition and an article molded therefrom may have excellent mixability and releasability.

Specifically, the recycled polyester resin composition may comprise a wax-based slip agent comprising at least one selected from the group consisting of fatty acid ester, fatty acid amide, polyethylene wax, vegetable wax, and mineral wax.

The content of the wax-based slip agent may be 0.001 part by weight to 0.2 part by weight relative to 100 parts by weight of the recycled polyester resin. For example, the content of the wax-based slip agent may be 0.002 part by weight to 0.2 part by weight, 0.005 part by weight to 0.2 part by weight, 0.01 part by weight to 0.18 part by weight, 0.02 part by weight to 0.15 part by weight, 0.05 part by weight to 0.15 part by weight, 0.05 part by weight to 0.12 part by weight, or 0.05 part by weight to 0.1 part by weight, relative to 100 parts by weight of the recycled polyester resin.

When the content of the wax-based slip agent satisfies the above range, the recycled polyester resin composition and an article molded therefrom may have excellent mixability and releasability. If the above range is exceeded, the volatility resistance of the recycled polyester resin composition and an article molded therefrom may be poor.

In addition, the recycled polyester resin composition may comprise at least one additive selected from the group consisting of flame retardants, nucleating agents, coupling agents, fillers, plasticizers, impact modifiers, lubricants, antibacterial agents, release agents, inorganic additives, ultraviolet stabilizers, antistatic agents, pigments, and dyes.

The recycled polyester resin composition may comprise the additive in an amount of 0.01 part by weight to 3 parts by weight relative to 100 parts by weight of the recycled polyester resin. For example, the total content of the additives may be 0.02 to 2.5 parts by weight, 0.03 part by weight to 2 parts by weight, 0.05 part by weight to 1.5 parts by weight, 0.1 part by weight to 1.2 parts by weight, 0.2 part by weight to 1 part by weight, or 0.5 part by weight to 1 part by weight.

**Molded Article**

The molded article according to another embodiment of the present invention is prepared from the recycled polyester resin composition.

As the molded article is prepared from the recycled polyester resin composition, it has excellent crystallinity, thermal resistance, and volatility resistance.

The molded article may be a molded article for a vehicle headlamp, but it is not limited thereto.

As a specific example, the molded article may be in the form of pellets obtained by melt-extruding the recycled polyester resin composition using a twin-screw extruder at a temperature of 200°C to 300°C or 220°C to 260°C.

**Process for preparing recycled dimethyl terephthalate**

The process for preparing recycled dimethyl terephthalate according to another embodiment of the present invention comprises depolymerizing waste polyester to obtain a depolymerization composition; and crystallizing the produced depolymerization composition.

The waste polyester may be obtained by crushing or melting waste polyester products. For example, the waste polyester may be obtained by crushing polyester products that have been used up and recovered and separated, or converting them into pellets (post-consumer recycled material; PCR), or polyester waste (post-industrial recycled material; PIR) such as defective products or scraps that may be formed during processes such as molding of polyester films, fibers, containers, or the like, but it is not limited thereto. As a specific example, the waste polyester may be waste polyethylene terephthalate (waste PET).

In addition, the waste polyester may be cut or crushed to sizes of 1 mm to 30 mm, 2 mm to 25 mm, or 3 mm to 20 mm to enhance process efficiency in the depolymerization step and may be prepared into recycled polyester chips through washing and drying.

For example, the waste polyester may have an intrinsic viscosity (IV) of greater than 0.3 dl/g, 0.35 dl/g or more, 0.4 dl/g or more, or 0.45 dl/g or more, and 1.1 dl/g or less, 1.0 dl/g or less, or 0.9 dl/g or less.

The process for preparing recycled dimethyl terephthalate according to another embodiment of the present invention comprises depolymerizing waste polyester to obtain a depolymerization composition.

The depolymerization may comprise alcoholysis. Specifically, the alcoholysis may be carried out using an alcohol, more specifically, a monoalcohol or a glycol. In addition, the depolymerization may comprise glycolysis. Specifically, the glycolysis may be carried out using ethylene glycol.

In addition, the depolymerization may be carried out at 80°C to 240°C. For example, the depolymerization may be carried out at 85°C to 235°C, 90°C to 225°C, or 90°C to 220°C.

According to an embodiment of the present invention, the depolymerization may be carried out in one step or in two steps of first depolymerization and second depolymerization.

As a specific example, the depolymerization may be carried out by alcoholysis of waste polyester or may be carried out by first depolymerizing waste polyester to produce recycled bis(2-hydroxyethyl) terephthalate (rBHET) and then second depolymerizing the rBHET. More specifically, the first depolymerization may be depolymerization through glycolysis using ethylene glycol, and the second depolymerization may be depolymerization through alcoholysis using an alcohol.

For example, the waste polyester and methanol are charged to a first high-pressure reactor, an alcoholysis catalyst is added thereto, and alcoholysis may be carried out while all connecting parts of the first high-pressure reactor are sealed.

As another example, the waste polyester, ethylene glycol, and zinc acetate anhydride may be charged to a stainless steel reactor, and first depolymerization through glycolysis may be carried out to obtain solid rBHET. Thereafter, the solid rBHET and methanol are charged to a first high-pressure reactor, an alcoholysis catalyst is added thereto, and the second depolymerization through alcoholysis may be carried out to obtain solid rDMT while all connecting parts of the first high-pressure reactor are sealed.

The alcoholysis may be carried out at a temperature of 80°C to 240°C and a pressure of 2 bar to 60 bar for 2 hours to 5 hours. For example, the alcoholysis may be carried out at a temperature of 80°C to 235°C, 85°C to 230°C, or 90°C to 230°C and a pressure of 2 bar to 60 bar, 3 bar to 60 bar, 4 bar to 60 bar, 3 bar to 20 bar, 3 bar to 15 bar, 35 bar to 60 bar, 40 bar to 60 bar, or 50 bar to 60 bar for 2.5 hours to 5 hours, 2.5 hours to 4 hours, or 3 hours to 5 hours.

An alcoholysis catalyst may be added for the alcoholysis. The alcoholysis can be smoothly carried out as a non-catalytic reaction without using an alcoholysis catalyst, making it environmentally friendly. In particular, when the content of insoluble metals in waste polyester is high, a non-catalytic reaction may be advantageous for efficient treatment and removal of impurities. In addition, an alcoholysis catalyst may be added from the viewpoint of energy to enhance processability by improving reactivity.

The alcoholysis catalyst may be a metal acetate salt, an alkali metal salt, or a hydroxy salt.

More specifically, the alcoholysis catalyst may comprise at least one cation selected from the group consisting of alkali metal ions such as $Li^+$, $Na^+$, $K^+$, and $Cs^+$, alkali earth metal ions such as $Be^{2+}$, $Mg^{2+}$, $Ca^{2+}$, and $Ba^{2+}$, ammonium ions such as $NH^{4+}$ and $NR^{4+}$ (where R is alkyl), and $Zn^{2+}$; or at least one anion selected from the group consisting of $OH^-$, $OR^-$ (where R is alkyl), $HCO_3^-$, $CO_3^{2-}$, benzoate ion ($C_2H_5O_2^-$), 4-alkoxycarbonylbenzoate ion, acetate ion, and terephthalate ion. R may be an alkyl group with 1 to 10 carbon atoms or an alkyl group with 1 to 5 carbon atoms.

For example, the alcoholysis catalyst may comprise at least one selected from the group consisting of $Zn(OAC)_2$, $Co(OAc)_2$, $Mn(OAc)_2$, $Mg(OAc)_2$, $Ca(OAc)_2$, $Ba(OAc)_2$, LiOAc, NaOAc, KOAc, $Zn(OAC)_2 \cdot 2H_2O$, $Co(OAc)_2 \cdot 4H_2O$, $Pb(OAc)_2$, $Mn(OAc)_2 \cdot 4H_2O$, $Mg(OAc)_2 \cdot 4H_2O$, $Pd(OAc)_2$, $Ti(OBu)_4$, $Ti(OiPr)_4$, $GeO_2$, $Al(OiPr)_3$, $Na_2CO_3$, $K_2CO_3$, dibutyltin(IV) oxide, stannous octoate, titanium phosphate, and terephthalic acid.

The amount of the alcoholysis catalyst added may be 10 ppm to 10,000 ppm based on the total weight of the waste polyester. For example, when the depolymerization is carried out in one step, the amount of the alcoholysis catalyst added may be 10 ppm to 9,000 ppm, 50 ppm to 8,000 ppm, 100 ppm to 6,000 ppm, 250 ppm to 3,500 ppm, 300 ppm to 2,000 ppm, 500 ppm to 1,500 ppm, or 600 ppm to 1,200 ppm, relative to the total weight of the waste polyester.

In addition, the amount of the alcoholysis catalyst added may be 10 ppm to 10,000 ppm relative to the total weight of the rBHET. For example, when the depolymerization is carried out in two steps, and when the depolymerization for the alcoholysis reaction is the second depolymerization, the amount of the alcoholysis catalyst added may be 10 ppm to 9,000 ppm, 50 ppm to 8,000 ppm, 100 ppm to 6,000 ppm, 250 ppm to 3,500 ppm, 300 ppm to 2,000 ppm, or 300 ppm to 1,500 ppm, relative to the total weight of the intermediate product produced through the first depolymerization, a specific example being rBHET.

In addition, the glycolysis may be carried out at 160°C to 240°C. For example, the glycolysis may be carried out by raising the temperature inside the reactor to 170°C to 220°C, 175°C to 210°C, or 185°C to 200°C.

The process for preparing recycled dimethyl terephthalate according to another embodiment of the present invention comprises crystallizing the produced depolymerization composition.

The crystallization step may be carried out by cooling the depolymerization composition to 25°C to 60°C and stirring it at a low speed of 10 rpm to 30 rpm or allowing it to stand for 1 hour to 6 hours. For example, the crystallization step may be carried out by cooling the depolymerization composition to 25°C to 55°C, 30°C to 60°C, or 40°C to 55°C and stirring it at a low speed of 10 rpm to 25 rpm or 10 rpm to 20 rpm or allowing it to stand for 1 hour to 5 hours or 2 hours to 4 hours.

More specifically, as the crystallization step under the above conditions is carried out after completion of the depolymerization, a depolymerization composition in the form of a slurry can be obtained. As a specific example, as the crystallization step under the above conditions is carried out after completion of the depolymerization by alcoholysis, an alcoholysis composition in the form of a slurry can be obtained. The depolymerization composition in the form of a slurry may comprise solid-phase recycled dimethyl terephthalate (rDMT), an excess amount of unreacted methanol, a small amount of liquid-phase dissolved recycled dimethyl terephthalate (rDMT), an ethylene glycol-based derivative produced by a side reaction, a polyester oligomer, a methanol-terminated oligomer, and the like.

In such an event, the depolymerization composition obtained through the crystallization step may comprise an ethylene glycol-based derivative produced by a side reaction or terephthalic acid and a terephthalate-based derivative produced other than the desired DMT or BHET, wherein the respective components and contents thereof may be analyzed using ultra-performance liquid chromatography (UPLC).

In addition, upon completion of the depolymerization, the components fed for depolymerization may be recovered through layer separation or fractional distillation. As a specific example, methanol fed for the alcoholysis or ethylene glycol fed for the glycolysis may be recovered through layer separation or fractional distillation.

In addition, an ethylene glycol-based derivative as a by-product may be produced in the depolymerization step.

The content of the ethylene glycol-based derivative may be 5% by weight or less based on the total weight of the recycled dimethyl terephthalate. For example, the ethylene glycol-based derivative may comprise ethylene glycol and diethylene glycol, and the content of the ethylene glycol-based derivative may be 4% by weight or less, 3.5% by weight or less, 3% by weight or less, 2.5% by weight or less, or 2% by weight or less, and 0.1% by weight or more, 0.2% by weight or more, 0.3% by weight or more, or 0.5% by weight or more, based on the total weight of the recycled dimethyl terephthalate.

In addition, terephthalic acid and a terephthalate-based derivative as a by-product may be produced in the depolymerization step.

The terephthalate-based derivative does not comprise dimethyl terephthalate while it may be at least one selected from the group consisting of dimethyl isophthalate (DMI), methyl hydrogen terephthalate (MHT), 1-(2-hydroxyethyl)4-methyl terephthalate (HEMT), ethylmethyl terephthalate (EMT), mono(hydroxylethyl) terephthalate (MHET), 2-hydroxyethyl [2-(2-hydroxyethoxy)ethyl] terephthalate (HEHDET), and bis[2-(2-hydroxyethoxy)ethyl] terephthalate (BDHET), but it is not limited thereto.

In such an event, the content of the terephthalic acid and terephthalate-based derivative may be 10% by weight or less based on the total weight of the recycled dimethyl terephthalate. For example, the content of the terephthalic acid and terephthalate-based derivative may be 9% by weight or less, 8% by weight or less, 6.5% by weight or less, 5% by weight or less, 3% by weight or less, 1.5% by weight or less, or 1% by weight or less, based on the total weight of the recycled dimethyl terephthalate.

In addition, the rDMT finally prepared through the depolymerization may comprise inorganic compounds. For example, the rDMT finally prepared may comprise at least one inorganic compound selected from the group consisting of antimony-containing compounds, germanium-containing compounds, titanium-containing compounds, zinc-containing compounds, cobalt-containing compounds, tin-containing compounds, aluminum-containing compounds, magnesium, sodium salts, potassium salts, phosphorus-containing compounds, and sulfur-containing compounds. The inorganic compounds may be derived from waste polyester and may be additional components added in the depolymerization process. The content of the inorganic compound may be 10 ppm to 500 ppm, 30 ppm to 400 ppm, 50 ppm to 350 ppm, or 100 ppm to 300 ppm, based on the total weight of the rDMT.

According to an embodiment of the present invention, the process for preparing recycled dimethyl terephthalate may further comprise at least one step selected from the group consisting of a solid-liquid separation step, a drying step, a washing step, and a purification step.

For example, a washing step and a purification step may be carried out after the crystallization step, and a solid-liquid separation step, a washing step, and a purification step may be carried out after the crystallization step.

More specifically, the solid-liquid separation step, the drying step, the washing step, and the purification step may be sequentially carried out after the crystallization step. As a specific example, the depolymerization composition obtained through the crystallization step may be separated into solid and liquid, and then dried and washed to obtain rDMT in the form of a wet cake. The washed rDMT in the form of a wet cake may be purified to obtain solid rDMT.

The solid-liquid separation may be carried out using a centrifuge. Specifically, the solid rDMT or rBHET contained in the depolymerization composition may be separated from the liquid components fed in the depolymerization, such as ethylene glycol and methanol, through the solid-liquid separation. Specifically, the solid-liquid separation may be carried out using a centrifuge at 1,000 rpm to 4,000 rpm, 1,500 rpm to 3,500 rpm, or 2,000 rpm to 3,000 rpm.

The drying step may be carried out at 85°C to 130°C. For example, the temperature at which the drying step is carried out may be 90°C to 120°C, 95°C to 110°C, or 95°C to 105°C.

The washing may be carried out three or more times using a mixture of an alcohol and/or water, a protic solvent such as isopropanol and acetic acid, or an aprotic solvent such as acetone, dichloromethane, chloroform, tetrahydrofuran (THF), and toluene. Residual pigments or yellow impurities generated by the decomposition of pigments during hydrolysis can be effectively removed through the washing, thereby enhancing color characteristics.

The purification step may be carried out by fractional distillation under the conditions of a temperature of 170°C to 240°C and a reduced pressure of 100 Torr or less. For example, the purification step may be carried out by fractional distillation under the conditions of a temperature of 175°C to 235°C, 180°C to 225°C, or 190°C to 220°C and a reduced pressure of 100 Torr or less, 80 Torr or less, or 60 Torr or less. In addition, the purification step may further comprise cooling to room temperature after the fractional distillation.

## Process for preparing a recycled polyester resin

The process for preparing a recycled polyester resin according to another embodiment of the present invention comprises depolymerizing waste polyester to prepare recycled dimethyl terephthalate (rDMT); mixing the rDMT with a glycol component and subjecting the mixture to an esterification reaction to prepare recycled bis(4-hydroxybutyl) terephthalate (rBHBT); and subjecting the rBHBT to a polycondensation reaction to prepare a recycled polyester resin, wherein in the recycled polyester resin, the crystallized area value is 20% or more when calculated according to the above Equation 1 for the crystallized region and the non-crystallized region obtained by separating the crystal diffraction peaks appearing at $2\theta$ values of 0° to 50° by the individual peak fitting method in an X-ray diffraction (XRD) analysis.

The step of depolymerizing waste polyester to prepare recycled dimethyl terephthalate is as described above.

Details on the glycol component are as described above. The rDMT forms a residue of bis(4-hydroxybutyl) terephthalate, or an oligomer thereof, through an esterification reaction with a glycol. More specifically, the rBHBT or an oligomer thereof may form the polymer chains of a polyester resin finally prepared.

The esterification reaction may be carried out at 165°C or higher, 170°C or higher, 180°C or higher, or 190°C or higher, and may be 225°C or lower, 220°C or lower, 215°C or lower, 210°C or lower, or 205°C or lower. For example, the esterification reaction may be carried out at a temperature of 170°C or higher to facilitate smooth removal of methanol as a by-product and may be carried out at a temperature lower than the boiling point of a glycol by 10°C to reduce loss of the glycol to be substituted. As a specific example, the esterification reaction between 1,4-butanediol and rDMT may be carried out at 170°C to 220°C.

In addition, the esterification reaction may be carried out in a pressurized state higher than normal pressure by 0 kg/cm$^2$ to 10 kg/cm$^2$ (0 mmHg to 7,355.6 mmHg), 0 kg/cm$^2$ to 5 kg/cm$^2$ (0 to 3,677.8 mmHg) or 0 kg/cm$^2$ to 2.0 kg/cm$^2$ (0 to 1,471.1 mmHg). In addition, the esterification reaction may be carried out for 1 hour to 24 hours, 1 hour to 10 hours, or 1 hour to 6 hours.

The polycondensation reaction may be carried out at a temperature of 150°C to 300°C, 200°C to 290°C, 240°C to 280°C, or 260°C to 280°C. In addition, the polycondensation reaction may be carried out at a reduced pressure of 0.01 mmHg to 600 mmHg, 0.05 mmHg to 300 mmHg, or 0.1 mmHg to 100 mmHg. In addition, the polycondensation reaction may be carried out for the required time until the desired intrinsic viscosity is reached. For example, it may be carried out for 1 hour to 24 hours, 1 hour to 10 hours, or 1 hour to 4 hours. As the process conditions for polycondensation satisfy the above ranges, a glycol as a by-product of the polycondensation reaction can be advantageously removed from the system.

At the beginning of the polycondensation reaction, the stirring speed may be set high. As the polycondensation reaction proceeds, when the stirring power is weakened due to the increase in the viscosity of the reactant or the temperature of the reactant rises above the set temperature, the stirring speed may be appropriately adjusted accordingly.

In addition, in the esterification reaction step and/or polycondensation reaction step, a glycol component or an acid component may be further added.

The glycol component may comprise at least one selected from the group consisting of 1,3-propanediol, 1,4-cyclohexanedimethanol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 2,3-butanediol, 3-methyl-1,5-pentanediol, 3-methyl-2,4-pentanediol, 1,6-hexanediol, 1,2-cyclohexanediol, 1,4-cyclohexanediol, diethylene glycol, polyethylene glycol, polypropylene glycol, polytetramethylene glycol, polyhexamethylene glycol, copolymers of ethylene oxide and tetrahydrofuran, ethylene oxide-adducted polypropylene glycol, polycarbonate diol, polyneopentyl glycol, poly-3-methylpentanediol, and poly-1,5-pentanediol.

The acid component may comprise at least one selected from the group consisting of adipic acid, sebacic acid, succinic acid, isodecylsuccinic acid, maleic acid, fumaric acid, glutaric acid, azelaic acid, 1,4-cyclohexanedicarboxylic acid, 1,3-cyclohexanedicarboxylic acid, terephthalic acid, isophthalic acid, naphthalenedicarboxylic acid, diphenyldicarboxylic acid, 4,4'-stilbenedicarboxylic acid, 2,5-furandicarboxylic acid, and 2,5-thiophenedicarboxylic acid.

In addition, a catalyst and/or a stabilizer may be further added in the esterification reaction and in the polycondensation reaction.

For example, the catalyst for an esterification reaction may be methylates of sodium and magnesium; acetates, borates, fatty acid salts, and carbonates of Zn, Cd, Mn, Co, Ca, and Ba; metallic Mg; and oxides of Pb, Zn, Sb, Ge, and Ti.

In addition, the catalyst for a polycondensation reaction may be, for example, titanium-based catalysts such as tetraethyl titanate, acetyl tripropyl titanate, tetrapropyl titanate, tetrabutyl titanate, polybutyl titanate, 2-ethylhexyl titanate, octylene glycol titanate, lactate titanate, triethanolamine titanate, acetyl acetonate titanate, ethyl acetoacetic ester titanate, isostearyl titanate, titanium dioxide, titanium dioxide/silicon dioxide copolymers, titanium dioxide/zirconium dioxide copolymers; germanium-based catalysts such as germanium dioxide and copolymers using the same; or tin-based catalysts such as monobutyltin oxide, dibutyltinoxide, and monobutylhydroxytinoxide.

In addition, the stabilizer may be a phosphorus-based compound such as phosphoric acid, trimethyl phosphate, and triethyl phosphate may be used, but it is not limited thereto.

The process for preparing a polyester resin according to another embodiment of the present invention may further comprise carrying out a solid-state polymerization reaction. For example, the solid-state polymerization may be carried out after the polycondensation reaction at a temperature of 190°C to 230°C under vacuum conditions of 0.2 Torr to 2.0 Torr or a nitrogen atmosphere.

## Process for preparing a recycled polyester resin composition

The process for preparing a recycled polyester resin composition according to another embodiment of the present invention comprises mixing a recycled polyester resin comprising a repeat unit derived from a glycol component and a repeat unit derived from recycled dimethyl terephthalate (rDMT); a hindered phenol-based primary antioxidant; and a phosphorus-based or sulfur-based secondary antioxidant.

According to another embodiment, a wax-based slip agent comprising at least one selected from the group consisting of fatty acid ester, fatty acid amide, polyethylene wax, vegetable wax, and mineral wax may be further added in the mixing step. According to another embodiment, at least one additive selected from the group consisting of flame retardants, nucleating agents, coupling agents, fillers, plasticizers, impact modifiers, lubricants, antibacterial agents, release agents, inorganic additives, ultraviolet stabilizers, antistatic agents, pigments, and dyes may be further added in the mixing step.

The amount of the respective components to be mixed may be appropriately adjusted according to the content in the recycled polyester resin composition exemplified above.

In addition, the recycled polyester resin composition may be processed into pellets after mixing. For example, the recycled polyester resin composition is fed to an extruder, volatile gases are removed at a reduced pressure, and the composition may be prepared into a pellet form using a chip cutter at about 200°C to 300°C.

## Mode for the Invention

Hereinafter, the present invention will be described in more detail with reference to the following examples. However, these examples are set forth to illustrate the present invention, and the scope of the present invention is not limited thereto.

## Preparation of recycled dimethyl terephthalate (rDMT)

## Preparation Example 1

A first high-pressure reactor with a capacity of 1 liter was charged with 120 g of waste polyethylene terephthalate (waste PET) and 400 g of methanol, followed by an addition thereto of 120 mg of $Zn(OAC)_2 \cdot 2H_2O$ as an alcoholysis catalyst (1,000 ppm relative to the total weight of the waste PET).

Thereafter, with all connections of the first high-pressure reactor tightened and sealed, the temperature was raised to 220°C over 1 hour. Depolymerization through alcoholysis was carried out by stirring for 3 hours while a temperature of 220°C and a pressure of 58 bar were maintained. Upon completion of the alcoholysis, the mixture was cooled to room temperature and stirred at a low speed of 10 rpm to 20 rpm for 2 hours to carry out crystallization, thereby obtaining an alcoholysis composition in the form of a slurry. In such an event, the alcoholysis composition comprised solid recycled dimethyl terephthalate (rDMT), excess unreacted methanol, and ethylene glycol derivatives produced by side reactions.

Thereafter, the alcoholysis composition was charged to a centrifuge and subjected to solid-liquid separation, dried in an oven at 100°C, and then washed with methanol three times or more to obtain rDMT in the form of a wet cake. The washed rDMT in the form of a wet cake was subjected to fractional distillation at a temperature of 205°C and a reduced pressure of 100 Torr or less and cooled to room temperature to obtain further purified solid rDMT. In such an event, the respective components and contents of ethylene glycol derivatives produced by side reactions or terephthalic acid and terephthalate-based derivatives produced other than the desired DMT were analyzed using ultra-performance liquid chromatography (UPLC) (see Table 2 below).

Thereafter, the filtrate remaining as a result of the filtration of the solid rDMT was charged to a separate flask, and the excess unreacted methanol and ethylene glycol (EG) formed by a side reaction were respectively recovered using a fractional distillation apparatus.

**Preparation Example 2**

A reactor made of stainless steel (SUS) was charged with 1,000 g of waste polyethylene terephthalate (waste PET), 4,000 g of ethylene glycol, and 3.5 g of zinc acetate anhydride. The temperature inside the reactor was raised to 196°C, and first depolymerization through glycolysis was carried out for 4 hours.

Thereafter, the first depolymerization composition was cooled to 30°C and stirred at 10 rpm to 20 rpm for 2 hours to carry out crystallization. As a result, a slurry comprising recycled bis(2-hydroxyethyl) terephthalate (rBHET) and an excess of ethylene glycol (EG) was obtained.

Thereafter, the slurry was charged to a centrifuge and subjected to solid-liquid separation, and washed twice with distilled water. The residual solvent was removed using an oven at 80°C to obtain about 1,000 g of solid rBHET. In such an event, the respective components and contents of ethylene glycol-based derivatives produced by side reactions or terephthalic acid and terephthalate-based derivatives produced other than the desired bis(2-hydroxyethyl) terephthalate (rBHET) were analyzed using ultra-performance liquid chromatography (UPLC) (see Table 2 below).

Thereafter, the filtrate remaining as a result of the filtration of the solid rBHET was charged to a separate flask, and the excess unreacted ethylene glycol (EG) was recovered using a fractional distillation apparatus.

Thereafter, a first high-pressure reactor with a capacity of 1 liter was charged with 150 g of the solid rBHET and 400 g of methanol, followed by an addition thereto of 45 mg of $Zn(OAC)_2 \cdot 2H_2O$ as an alcoholysis catalyst (300 ppm relative to the total weight of the rBHET).

Thereafter, with all connections of the first high-pressure reactor tightened and sealed, the temperature was raised to 90°C over 1 hour. The second depolymerization through alcoholysis was carried out by stirring for 5 hours while a temperature of 90°C and a pressure of 3 bar were maintained. Upon completion of the alcoholysis, the mixture was cooled to room temperature and allowed to stand for 4 hours, thereby obtaining a second depolymerization composition in the form of a slurry. In such an event, the second depolymerization composition comprised solid recycled dimethyl terephthalate (rDMT), excess unreacted methanol, and ethylene glycol derivatives produced by side reactions.

Thereafter, the second depolymerization composition was charged to a centrifuge and subjected to solid-liquid separation, dried in an oven at 100°C, and then washed with methanol three times or more to obtain rDMT in the form of a wet cake. The washed rDMT in the form of a wet cake was subjected to fractional distillation at a temperature of 205°C and a reduced pressure of 100 Torr or less and cooled to room temperature to obtain further purified solid rDMT. In such an event, the respective components and contents of ethylene glycol-based derivatives produced by side reactions or terephthalic acid and terephthalate-based derivatives produced other than the desired DMT were analyzed using ultra-performance liquid chromatography (UPLC) (see Table 2 below).

Thereafter, the filtrate remaining as a result of the filtration of the solid rDMT was charged to a separate flask, and the excess unreacted methanol and ethylene glycol (EG) formed by a side reaction were respectively recovered using a fractional distillation apparatus.

**Preparation Example 3**

A reactor made of stainless steel (SUS) was charged with 1,000 g of waste polyethylene terephthalate (waste PET), 4,000 g of ethylene glycol, and 3.5 g of zinc acetate anhydride. The temperature inside the reactor was raised to 196°C, and first depolymerization through glycolysis was carried out for 4 hours.

Thereafter, the first depolymerization composition was cooled to 30°C and stirred at 10 rpm to 20 rpm for 2 hours to carry out crystallization. As a result, a slurry comprising recycled bis(2-hydroxyethyl) terephthalate (rBHET) and an excess of ethylene glycol (EG) was obtained.

Thereafter, the slurry was charged to a centrifuge and subjected to solid-liquid separation, and washed twice with distilled water. The residual solvent was removed using an oven at 80°C to obtain about 1,000 g of solid rBHET. In such an event, the respective components and contents of ethylene glycol-based derivatives produced by side reactions or terephthalic acid and terephthalate-based derivatives produced other than the desired bis(2-hydroxyethyl) terephthalate (rBHET) were analyzed using ultra-performance liquid chromatography (UPLC) (see Table 2 below).

Thereafter, the filtrate remaining as a result of the filtration of the solid rBHET was charged to a separate flask, and the excess unreacted ethylene glycol (EG) was recovered using a fractional distillation apparatus.

Thereafter, a first high-pressure reactor with a capacity of 1 liter was charged with 150 g of the solid rBHET and 400 g of methanol, followed by an addition thereto of 45 mg of $Zn(OAC)_2 \cdot 2H_2O$ as an alcoholysis catalyst (300 ppm relative to the total weight of the rBHET).

Thereafter, with all connections of the first high-pressure reactor tightened and sealed, the temperature was raised to 220°C over 1 hour. The second depolymerization through alcoholysis was carried out by stirring for 3 hours while a temperature of 220°C and a pressure of 58 bar were maintained. Upon completion of the alcoholysis, the mixture was cooled to room temperature and allowed to stand for 3 hours, thereby obtaining a second depolymerization composition in the form of a slurry. In such an event, the second depolymerization composition comprised solid recycled dimethyl terephthalate (rDMT), excess unreacted methanol, and ethylene glycol derivatives produced by side reactions.

Thereafter, the second depolymerization composition was charged to a centrifuge and subjected to solid-liquid separation and dried in an oven at 100°C to obtain solid rDMT. In such an event, the respective components and contents of ethylene glycol-based derivatives produced by side reactions or terephthalic acid and terephthalate-based derivatives produced other than the desired DMT were analyzed using ultra-performance liquid chromatography (UPLC) (see Table 2 below).

Thereafter, the filtrate remaining as a result of the filtration of the solid rDMT was charged to a separate flask, and the excess unreacted methanol and ethylene glycol (EG) formed by a side reaction were respectively recovered using a fractional distillation apparatus.

**Preparation Example 4**

A reactor made of stainless steel (SUS) was charged with 1,000 g of waste polyethylene terephthalate (waste PET), 4,000 g of ethylene glycol, and 3.5 g of zinc acetate anhydride. The temperature inside the reactor was raised to 196°C, and first depolymerization through glycolysis was carried out for 4 hours.

Thereafter, the first depolymerization composition was cooled to 30°C and stirred at 10 rpm to 20 rpm for 2 hours to carry out crystallization. As a result, a slurry comprising recycled bis(2-hydroxyethyl) terephthalate (rBHET) and an excess of ethylene glycol (EG) was obtained.

Thereafter, the slurry was charged to a centrifuge and subjected to solid-liquid separation, and washed twice with distilled water. The residual solvent was removed using an oven at 80°C to obtain about 1,000 g of solid rBHET. In such an event, the respective components and contents of ethylene glycol-based derivatives produced by side reactions or terephthalic acid and terephthalate-based derivatives produced other than the desired bis(2-hydroxyethyl) terephthalate (rBHET) were analyzed using ultra-performance liquid chromatography (UPLC) (see Table 2 below).

Thereafter, a first high-pressure reactor with a capacity of 1 liter was charged with 150 g of the solid rBHET and 400 g of methanol, followed by an addition thereto of 150 mg of $Zn(OAC)_2 \cdot 2H_2O$ as an alcoholysis catalyst (1,000 ppm relative to the total weight of the rBHET).

Thereafter, with all connections of the first high-pressure reactor tightened and sealed, the temperature was raised to 220°C over 1 hour. The second depolymerization through alcoholysis was carried out by stirring for 3 hours while a temperature of 220°C and a pressure of 58 bar were maintained. Upon completion of the alcoholysis, the mixture was cooled to room temperature and allowed to stand for 3 hours, thereby obtaining a second depolymerization composition in the form of a slurry. In such an event, the second depolymerization composition comprised solid recycled dimethyl terephthalate (rDMT), excess unreacted methanol, and ethylene glycol derivatives produced by side reactions.

Thereafter, the second depolymerization composition was subjected to a first fractional distillation under the conditions of 60°C and 500 mmHg, a second fractional distillation under the conditions of 170°C and 300 mmHg, and a third fractional distillation under the conditions of 200°C and 60 mmHg to obtain a solid rDMT. In such an event, the respective components and contents of ethylene glycol-based derivatives produced by side reactions or terephthalic acid and terephthalate-based derivatives produced other than the desired DMT were analyzed using ultra-performance liquid chromatography (UPLC) (see Table 2 below).

Thereafter, the filtrate remaining as a result of the filtration of the solid rDMT was charged to a separate flask, and the excess unreacted methanol and ethylene glycol (EG) formed by a side reaction were respectively recovered using a fractional distillation apparatus.

**Test Example 1-1: Ultra-performance liquid chromatography (UPLC)**

Ultra-performance liquid chromatography (UPLC) was used to measure the purity of the rDMT obtained in Preparation Examples 1 to 4 and the rBHET obtained in Preparation Example 2 and to analyze the components and contents of ethylene glycol derivatives produced by side reactions or terephthalic acid or terephthalate derivatives produced other than the desired DMT or BHET.

Specifically, according to the mobile phase and flow rate in Table 1 below, 0.01 g of a sample was diluted in 20 ml of a mixture of methanol and 0.3% phosphoric acid solution in distilled water. The area of a peak was measured using ultra-performance liquid chromatography (manufacturer: Water, model: ACQUITY UPLC H-Class Systems), and its fraction (%) relative to the total peak area was then calculated to determine the purity, the components formed by side reactions, the components formed other than the target DMT, and their contents. In such an event, commercially available DMT (manufacturer: SK Chemicals, model name: SKYDMT) was shown as a control group in Table 2.

[Table 1]

| Time (min.) | Flow (ml/min.) | 0.3% $H_3PO_4$ in DW (%) | Methanol (%) |
|---|---|---|---|
| 0.0 | 0.2 | 60 | 40 |
| 1.0 | 0.2 | 60 | 40 |
| 4.5 | 0.2 | 27.5 | 72.5 |
| 6.5 | 0.2 | 27.5 | 72.5 |
| 7.5 | 0.2 | 60 | 40 |
| 12.5 | 0.2 | 60 | 40 |

**Test Example 1-2: APHA color values**

The rDMT obtained in Preparation Examples 1 to 4 and the rBHET obtained in Preparation Example 2 were measured for APHA (American Public Health Association) color values according to ASTM-D1209. Specifically, according to ASTM-D1209, the rDMT and the rBHET were each dissolved in a dimethylformamide solvent at a concentration of 10% by weight and placed in an analysis vessel made of quartz and having a light path length of 10 mm. The APHA color value was measured using the transmittance mode of a colorimeter (model name: ColorEye7000A).

[Table 2]

| | | DMT (control group) | rDMT | | | | rBHET |
|---|---|---|---|---|---|---|---|
| | | | Prep. Ex. 1 | Prep. Ex. 2 | Prep. Ex. 3 | Prep. Ex. 4 | Intermediate product in Prep. Ex. 2 |
| Component and content (ppm) | BHET | - | 10 | 41,230 | 119,384 | 303,820 | 767,800 |
| | MHT | < 20 | 1,438 | 575 | 682 | 423 | - |
| | HEMT | - | 368 | 336 | 434 | 319 | - |
| | EMT | < 10 | 43 | 30 | 32 | 37 | - |
| | TPA | - | 10 | 3 | 5 | 11 | 11 |
| | EG | - | 11 | 261 | 403 | 10,082 | 689 |
| | DEG | - | 11 | 100 | 128 | 450 | 582 |
| | MHET | - | - | - | - | - | 69,300 |
| | HEHDET | - | - | - | - | - | 119,800 |
| | BDHET | - | - | - | - | - | 8,300 |
| Purity (%) of DMT or rDMT | | 100 | 99.811 | 95.747 | 87.893 | 68.486 | 3.352 |
| APHA | | 8 | 55 | 82 | 53 | 125 | 15 |

As can be seen from Table 2 above, the rDMT of Preparation Examples 1 and 2 prepared according to an embodiment of the present invention had a high purity of 95% or higher and excellent transparency with an APHA color value of 85 or lower. In particular, Preparation Examples 1 and 2 showed excellent purity and crystallinity due to low contents of ethylene glycol and diethylene glycol. They were suitable for use as raw materials for various polymers since they had low contents of terephthalate derivatives such as methyl hydrogen terephthalate (MHT) other than the target DMT.

In addition, Preparation Example 3, which did not perform the washing process using methanol, showed a low purity of less than 90%. Preparation Example 4, which performed the conventional fractional distillation process, showed a very low transparency with an APHA color value of 125, and the purity was also very low at approximately 68%.

**Preparation of a recycled polyester resin**

**Resin A**

Step (1): Preparation of recycled bis(4-hydroxybutyl) terephthalate (rBHBT) through a transesterification reaction

A 1-liter reactor for a transesterification reaction equipped with a column and a condenser that can be cooled by water was charged with 3,068 g of 1,4-butanediol (BD) as a glycol component, 4,407 g of rDMT prepared in Preparation Example 1 as an acid component, and 2 g of tetrabutyl titanate (TBT) as a transesterification reaction catalyst. The pressure in the reactor was adjusted to 0.1 kg/cm$^2$ by flowing nitrogen, and the temperature was raised while the pressure was maintained with stirring. A transesterification reaction was carried out while the temperature was maintained at 200°C. In such an event, methanol as a by-product was discharged through the column and the condenser during the transesterification reaction. The transesterification reaction was carried out until the discharge of methanol stopped, thereby obtaining a transesterification reaction composition comprising rBHBT and its oligomers. Upon completion of the transesterification reaction, nitrogen in the pressurized reactor was released to the outside to lower the pressure in the reactor to normal pressure.

Step (2): Preparation of a recycled polyester resin through a polycondensation reaction

The transesterification reaction composition obtained in step (1) was charged to a polycondensation reactor. Thereafter, the pressure of the reactor was reduced from normal pressure to 5.0 Torr (absolute pressure: 5 mmHg) over 30 minutes. At the same time, the temperature of the reactor was raised to 245°C over 1 hour, and a polycondensation reaction was carried out while the pressure of the reactor was maintained at 1.0 Torr (absolute pressure: 1.0 mmHg) or less. In such an event, at the beginning of the polycondensation reaction, the stirring speed was set high. As the polycondensation reaction proceeded, the glycol component as a by-product was discharged from the reactor. When the stirring power was weakened due to the increase in the viscosity of the mixture in the reactor or the temperature of the mixture was elevated above the set temperature, the stirring speed was appropriately adjusted. The polycondensation reaction was carried out until the intrinsic viscosity (IV) of the mixture (melt) in the reactor reached 1.14 dl/g. When the intrinsic viscosity of the mixture in the reactor reached the desired level, the mixture was then discharged to the outside of the reactor to form strands, which were solidified with a cooling liquid and then granulated to have an average weight of about 12 mg to 14 mg to prepare a polyester resin. As a result, 5,000 g of a recycled polyester (rPBT) resin was obtained.

**Resin B**

A transesterification reaction was carried out in the same manner as in Example 1, except that, in step (1), rDMT prepared in Preparation Example 2 was used instead of rDMT prepared in Preparation Example 1. A polycondensation was carried out in the same manner as in Example 1 until the intrinsic viscosity (IV) in the reactor reached 0.98 dl/g in step (2), thereby obtaining 5,000 g of a recycled polyester (rPBT) resin.

**Resin C**

A polycondensation was carried out in the same manner as in Example 1 until the intrinsic viscosity (IV) in the reactor reached 0.72 dl/g in step (2), thereby obtaining 5,000 g of a recycled polyester (rPBT) resin.

**Resin D**

A transesterification reaction was carried out in the same manner as in Example 1, except that, in step (1), commercially available DMT (manufacturer: SK Chemicals, model name: SKYDMT) was used instead of rDMT prepared in Preparation Example 1. A polycondensation was carried out in the same manner as in Example 1 until the intrinsic viscosity (IV) in the reactor reached 1.11 dl/g in step (2), thereby obtaining 5,000 g of a virgin polyester (PBT) resin.

**Resin E**

A transesterification reaction was carried out in the same manner as in Example 1, except that, in step (1), rDMT prepared in Preparation Example 3 was used instead of rDMT prepared in Preparation Example 1. A polycondensation was carried out in the same manner as in Example 1 until the intrinsic viscosity (IV) in the reactor reached 1.1 dl/g in step (2), thereby obtaining 5,000 g of a recycled polyester (rPBT) resin.

**Resin F**

A transesterification reaction was carried out in the same manner as in Example 1, except that, in step (1), rDMT prepared in Preparation Example 4 was used instead of rDMT prepared in Preparation Example 1. A polycondensation was carried out in the same manner as in Example 1 until the intrinsic viscosity (IV) in the reactor reached 0.9 dl/g in step (2), thereby obtaining 5,000 g of a recycled polyester (rPBT) resin.

**Resin G**

A transesterification reaction was carried out in the same manner as in Example 1, except that, in step (1), 2,836 g of 1,4-butanediol (BD) as a glycol component and 2,852 g of rDMT prepared in Preparation Example 4 and 1,600 g of rBHET prepared in Preparation Example 2 as acid components were used. A polycondensation was carried out in the same manner as in Example 1 until the intrinsic viscosity (IV) in the reactor reached 0.83 dl/g in step (2), thereby obtaining 5,000 g of a recycled polyester (rPBT) resin.

**Resin H**

A transesterification reaction was carried out in the same manner as in Example 1, except that, in step (1), 1,783 g of 1,4-butanediol (BD) as a glycol component and 6,287 g of rBHET prepared in Preparation Example 2 as an acid component were used. A polycondensation was carried out in the same manner as in Example 1 until the intrinsic viscosity (IV) in the reactor reached 0.81 dl/g in step (2), thereby obtaining 5,000 g of a recycled polyester (rPBT) resin.

**Resin I**

A transesterification reaction was carried out in the same manner as in Example 1, except that, in step (1), 1,286 g of 1,4-butanediol (BD) as a glycol component and 7,257 g of rBHET prepared in Preparation Example 2 as an acid component were used. A polycondensation was carried out in the same manner as in Example 1 until the intrinsic viscosity (IV) in the reactor reached 0.78 dl/g in step (2), thereby obtaining 5,000 g of a recycled polyester (rPBT) resin.

**Test Example 2-1: WAXD (wide angle X-ray diffraction) analysis**

Resins A to I were each taken for a WAXD plate photograph using a SmartLab type X-ray diffractometer from Rigaku. In such an event, the photographing conditions were as follows.

- X-ray generator: 3 kW (Cu target)
- Theta-theta goniometer with horizontal sample mounting
- Detector: D/teX Ultra250
- Application software: PDXL
- ICDD database PDF-2

For the WAXD analysis, correction data was used in which a polarization factor, an absorption factor, and an air scattering correction were performed for the measured data. Deconvolution was performed by single line fitting using the fundamental parameter (FP) approach of the fitting program (Bruker TOPAS) to separate the crystallized region peak and the non-crystallized region peak at 2θ values of 0° to 50° from the WAXD pattern derived from the X-ray diffraction (XRD) analysis, and the area and the area ratio of each peak were calculated (see Figs. 2 to 6). The crystallized area value (%) was calculated for the area of each obtained peak according to the following Equation 1.

Crystallized area value (%) = (area of crystallized region)/(area of crystallized region + area of non-crystallized region) $\times$ 100          [Equation 1]

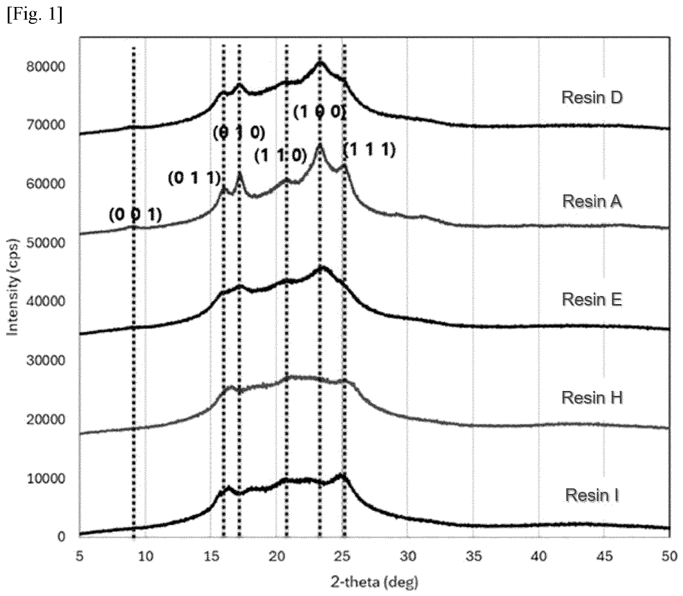

Fig. 1 shows X-ray diffraction (XRD) analyses of resins A, D, E, H, and I. As shown in Fig. 1, the recycled polyester resin A showed strong diffraction peaks due to crystals at angles (2θ, Braggs angles) of 9, 15.9, 17.2, 20.6, 23.4, and 25.1. These correspond to the diffraction planes (001), (011), (010), (110), (100), and (111) and correspond to the α crystal form of PBT. However, the recycled polyester resins prepared in Comparative Examples 2, 5, and 6 had low crystallinity since the positions of the peak angles corresponding to each diffraction plane shifted, or the areas of the peaks decreased.

## Test Example 2-2: Color-b and color-L

Resins A to I were each measured for color-b values and color-L values, which are color characteristics, using a colorimeter.

Specifically, the recycled polyester resins were each prepared into a specimen with a width of 30 mm, a length of 30 mm, and a thickness of 6 mm using a hot press. Transmission data was obtained with Illuminant D65 at an observer angle of 2°, and the data was processed using a color analysis device in Grams/32 software to measure the color-b value and the color-b value of the Hunter Lab.

## Test Example 2-3: Intrinsic viscosity

Resins A to I were each dissolved at a concentration of 0.12% in orthochlorophenol (OCP) at 150°C to obtain a solution, and an Ubbelohde viscometer was used in a constant temperature bath at 35°C to measure intrinsic viscosity. Specifically, the temperature of the viscosity tube was maintained at 35°C, and the time (efflux time) required for the solvent to pass between specific internal sections of the viscosity tube and the time required for the solution to pass were measured to obtain specific viscosity, which was used to calculate intrinsic viscosity.

## Test Example 2-4: Tm and ΔHm

Resins A to I were each measured for melting point (Tm) and melting enthalpy (ΔHm) using a differential scanning calorimeter (DSC, TA Instruments).

Specifically, each recycled polyester resin was dried under a reduced pressures at 50°C for 15 hours, melted at 260°C, then rapidly cooled to 30°C, and scanned at a temperature elevation rate of 10°C/minute using a differential scanning calorimeter. In such an event, melting enthalpy may be measured by using a differential scanning calorimeter by a first scan (1st scan) or a second scan (2nd scan). In the Test Example, it is measured by a second scan.

In the heat flow curve obtained by scanning, the first endothermic temperature is a glass transition temperature (Tg), the exothermic temperature measured after the glass transition temperature (Tg) is a crystallization temperature (Tc), and the endothermic temperature measured after the crystallization temperature (Tc) is a melting point (Tm). In addition, the integral value at the melting point (Tm) was calculated as melting enthalpy (ΔHm).

## Test Example 2-5: Heat deflection temperature (HDT) (1)

Resins A to I were each prepared into a specimen according to ASTM D648 and measured for heat deflection temperature at a low load of 0.48 MPa using a 6M-2 model tester from Toyoseiki.

## Test Example 2-6: $^1$H-NMR

Resins A to I were each dissolved in a CDCl$_3$ solvent at a concentration of 3 mg/ml, whose $^1$H-NMR spectrum was obtained at 25°C using nuclear magnetic resonance equipment (JEOL, 600MHz FT-NMR). The total content (% by mole) of an ethylene glycol residue and a diethylene glycol residue based on the total number of moles of residues derived from the entire glycols (EG, DEG, TEG, BD, and the like) was calculated by analyzing the $^1$H-NMR spectra.

[Table 3]

| | Resin A | Resin B | Resin C | Resin D | Resin E | Resin F | Resin G | Resin H | Resin I |
|---|---|---|---|---|---|---|---|---|---|
| DMT (g) | - | - | - | 4,407 | - | - | - | - | - |
| rDMT1 (g) | 4,407 | - | 4,661 | - | - | - | - | - | - |
| rDMT2 (g) | - | 4,407 | - | - | - | - | - | - | - |
| rDMT3 (g) | - | - | - | - | 4,407 | - | - | - | - |

(continued)

| | | Resin A | Resin B | Resin C | Resin D | Resin E | Resin F | Resin G | Resin H | Resin I |
|---|---|---|---|---|---|---|---|---|---|---|
| rDMT4 (g) | | - | - | - | - | - | 4,478 | 2,852 | - | - |
| rBHET (g) | | - | - | - | - | - | - | 1,600 | 6,287 | 7,257 |
| BD (g) | | 3,068 | 3,068 | 2,812 | 3,068 | 3,068 | 2,700 | 2,836 | 1,783 | 1,286 |
| Catalyst (TBT, g) | | 2 | 2 | 2 | 2 | 2 | 2 | 2 | 2 | 2 |
| Total amount added (g) | | 7,477 | 7,477 | 7,475 | 7,477 | 7,477 | 7,178 | 7,288 | 8,072 | 8,545 |
| Amount of P (g) | | 5,000 | 5,000 | 5,000 | 5,000 | 5,000 | 5,000 | 5,000 | 5,000 | 5,000 |
| Purity (%) of rDMT | | 99.8 | 95.7 | 99.8 | 100.0 | 87.9 | 68.5 | rBHET를 사용함 | | |
| EG and DEG (% by mole) | | 0.0 | 1.8 | 0.0 | 0.0 | 14.0 | 21.0 | 36.0 | 51.0 | 62.0 |
| IV (dl/g) | | 1.14 | 0.98 | 0.72 | 1.16 | 1.10 | 0.90 | 0.83 | 0.81 | 0.78 |
| Eq. 1 | 33.7 | 27.3 | 34.1 | 21.5 | 16.8 | 15.3 | 14.1 | 12.6 | 10.1 | 10.1 |
| | -12.2 | -5.8 | -12.7 | 0 | 4.7 | 6.1 | 7.3 | 8.8 | 11.4 | 11.4 |
| Color-b | | 7.0 | 8.1 | 7.0 | 2.1 | 12.8 | 18.1 | 17.4 | 18.9 | 19.2 |
| Color-L | | 83.0 | 81.4 | 83.0 | 88.0 | 68.2 | 63.5 | 66,3 | 61.3 | 62.7 |
| Tm (°C) | | 224.1 | 222.4 | 224.5 | 225.2 | 208.6 | 188.7 | 182.1 | 175.1 | 177.6 |
| ΔHm (J/g) | | 43.2 | 41.3 | 43.5 | 47.0 | 37.0 | 28.3 | 26.9 | 23.7 | 23.1 |
| HDT (°C) | | 99.3 | 98.4 | 101.2 | 97.4 | 81.8 | 62.8 | 56.1 | 49.0 | 52.9 |
| * rDMT1 to rDMT4 are rDMT prepared in Preparation Examples 1 to 4, respectively. | | | | | | | | | | |

As can be seen from Table 3 above, since Resins A to C were each prepared using rDMT with excellent purity, crystallinity, and color characteristics, they were excellent in such properties as color such as color-b and color-L, melting point (Tm), melting enthalpy, and heat deflection temperature, as well as crystallinity.

More specifically, Resins A to C satisfied a crystallized area value of 20% or more according to Equation 1, along with a difference in the crystallized area value of 5.8 or more according to Equation 3 when compared with Resin D in which commercially available dimethyl terephthalate, rather than recycled dimethyl terephthalate, was used, indicating excellent crystallinity. In addition, Resins A to C had a total content of an ethylene glycol residue and a diethylene glycol residue of 5% by mole or less based on the total number of moles of the glycols and their derivatives, indicating excellent purity and crystallinity.

In addition, Resins E, F, and G, in which rDMT having a purity of less than 90% was used alone, or together with rBHET, as an acid component and Resins H and I, in which rBHET was used alone, had a crystallized area value of 20% or less according to Equation 1, which was lower in crystallinity than Resin D, in which commercially available dimethyl terephthalate, rather than recycled dimethyl terephthalate, was used. As a result, Resins G to I were inferior in such properties as color such as color-b and color-L, melting point (Tm), melting enthalpy, and heat deflection temperature.

**Preparation of recycled polyester resin pellets**

**Examples 1 to 5 and Comparative Examples 1 to 8**

The components shown in Table 4 below were uniformly mixed as shown in Tables 5 and 6 below to prepare a resin composition. The resin composition was automatically metered and fed to the hopper of a twin-screw extruder (40 mm extruder, L/D: 40), volatile gases were removed at a reduced pressure, and the composition was prepared into a pellet form using a chip cutter at about 250°C. In such an event, information on the components used in the Examples and Comparative Examples is shown in Table 4 below. The values shown in Tables 5 and 6 below refer to parts by weight.

[Table 4]

| Hindered phenol-based antioxidant | 1-1010 | Pentaerythritol tetrakis(3-(3,5-di-tert-butyl-4-hydroxyphenyl)propionate), CAS No. 6683-19-8 |
|---|---|---|
| Hindered phenol-based antioxidant | 1-1098 | N,N'-(hexane-1,6-diyl)bis[3-(3,5-di-tert-butyl-4-hydroxyphe nyl)propanamide], CAS No. 23128-74-7 |
| Sulfur-based antioxidant | AO-412S | 2,2-Bis[[3-(dodecylthio)-1-oxopropoxy]methyl]propane-1,3-diyl Bis[3-(dodecylthio)propionate], CAS No. 29598-76-3 |
| Phosphorous-based antioxidant | 1-168 | Tris-(2,4-di-tert-butyl-phenyl)-phosphite, CAS No. 31570-04-4 |
| Wax-based slip agent | Sodium montanate | Sodium montanate, CAS No. 25728-82-9 |
| Wax-based slip agent | Incroslip SL | CRODA, CAS No. confidential |

[Table 5]

|  | Ex. 1 | Ex. 2 | Ex. 3 | Ex. 4 | Ex. 5 | Ex. 6 |
|---|---|---|---|---|---|---|
| Resin | A 100 | B 100 | C 100 | B+D 50:50 | C+D 50:50 | A 100 |
| 1-1010 | - | 0.05 | 0.01 | 0.05 | 0.05 | - |
| 1-1098 | 0.10 | - |  | - | - | 0.25 |
| AO-412S | - | 0.05 |  | - | 0.03 | - |
| 1-168 | 0.10 | - | 0.01 | 0.05 | - | 0.18 |
| Sodium montanate | - | 0.08 |  | - | - | - |
| Incroslip SL | 0.10 | - | 0.09 | 0.05 | 0.05 | - |

[Table 6]

|  | C. Ex. 1 | C. Ex. 2 | C. Ex. 3 | C. Ex. 4 | C. Ex. 5 | C. Ex. 6 | C. Ex. 7 |
|---|---|---|---|---|---|---|---|
| Resin | D 100 | E 100 | F 100 | G 100 | H 100 | I 100 | D+H 50:50 |
| 1-1010 | - | - | - | 0.05 | 0.40 | - | 0.05 |
| I-1098 | - | 0.05 | - | - | - | 0.35 | - |
| AO-412S | - | 0.05 | - | 0.03 | - | 0.35 | - |
| 1-168 | - | - | - | - | 0.35 | - | 0.05 |
| Sodium montanate | 0.05 | - | - | - | - | - | - |
| Incroslip SL | - | 0.30 | - | - | - | 0.05 | 0.05 |

**Test Example 3-1: Heat deflection temperature (HDT) (2)**

The recycled polyester resin pellets prepared in Examples 1 to 6 and Comparative Examples 1 to 7 were each prepared into a specimen according to ASTM D648, which specimen was aged and crystallized in a hot air oven at 110°C for 12 hours and then measured for heat deflection temperature at a low load of 0.45 MPa using a 6M-2 model tester from Toyoseiki.

Test Example 3-2: **Melt flow index (MI)**

The recycled polyester resin pellets prepared in Examples 1 to 6 and Comparative Examples 1 to 7 were each measured for a melt flow index (MI) under the conditions of 250°C and 2.16 kg according to ASTM D1238.

**Test Example 3-3: Tensile strength**

The recycled polyester resin pellets prepared in Examples 1 to 6 and Comparative Examples 1 to 7 were each measured for tensile strength (MPa) according to ASTM D638.

**Test Example 3-4: Evaluation of TVOC (total volatile organic content)**

The recycled polyester resin pellets prepared in Examples 1 to 6 and Comparative Examples 1 to 7 were each quantified for total volatile organic content according to the head space-gas chromatography/mass spectrometry (HS-GC/MS) analysis method.

(1) Sample pretreatment
About 0.1 g of a sample was placed in an HS vial (20 ml), seal with a clamp, and measured.
(2) HS (head space) device conditions

(i) Model: Triplus 500 (Thermo)
(ii) Incubation temperature: 130°C
(iii) Incubation time: 300 min.
(iv) Loop temperature: 200°C
(v) Loop volume: 1 ml
(vi) Injection time: 0.5 min.
(vii) Injection mode: standard

(3) GC/MS device conditions

(i) Model: Thermo 13010/ISQ7000
(ii) Column: DB-5MS (60 m $\times$ 0.32 mm $\times$ 1.0 $\mu$m)
(iii) Oven temp.: 40°C (5 min.) - 10 °C/min. - 300 (5 min.)
(iv) Loop temperature: 200°C
(v) Split ratio: 1:30
(vi) Flow rate: 1.5 ml/min. (constant flow)
(vii) Ion source & transfer line temp.: 250, 250°C
(viii) Mass range: 29-300

**Test Example 3-5: Fogging test**

The recycled polyester resin pellets prepared in Examples 1 to 6 and Comparative Examples 1 to 7 were each subjected to a fogging test according to the DIN 75201-A test method.
Specifically, 15 g of the pellets were placed in a glass chalet (thickness: 2 mm) with a diameter of 100 mm and a height of 20 mm, covered with a glass chalet (thickness: 2 mm) with a diameter of 120 mm and a height of 20 mm, and then heated on a hot plate at 130°C for 5 hours. The haze values of the glass chalet used as the cover were measured before and after heating to calculate the change in haze due to the volatile gases deposited on the glass plate (|haze value before heating - haze value after heating|). Fogging evaluation was performed based thereon. In such an event, the haze (%) was measured using NDH5000 from Nippon Denshoku. The change in haze was calculated according to the following Equation 2.

$$[\text{Equation 2}]$$

$$\text{Change in haze} = |H1 - H2|$$

In Equation 2, H1 is the haze measured before fogging evaluation under the conditions of 130°C and 5 hours, which is a numerical value excluding the unit, and H2 is the haze due to gas formed during fogging evaluation under the conditions of 130°C and 5 hours, which is a numerical value excluding the unit.

**Test Example 3-6: Evaluation of oxidative induction time (OIT)**

The recycled polyester resin pellets prepared in Examples 1 to 6 and Comparative Examples 1 to 7 were each measured for the time at which the oxidation exothermic reaction occurred to evaluate the oxidation induction time.

Specifically, the pellets were dried under a reduced pressure at 50°C for 15 hours and then heated at 10°C/minute from 30°C to 240°C using a differential scanning calorimeter (DSC, TA Instruments) in a nitrogen atmosphere. Thereafter, the conditions were changed from nitrogen to air at a conversion rate of 20 ml/minute, and the time at which the oxidation exothermic reaction took place was measured while 240°C was maintained for 60 minutes.

**Test Example 3-7: Injection flow mark**

The recycled polyester resin pellets prepared in Examples 1 to 6 and Comparative Examples 1 to 7 were each injection molded to a plate having a size of 120 mm × 120 mm × 2 T to prepare a molded article. The surface appearance of the molded article was visually observed, and whether or not a striped flow mark was observed was evaluated as × or ∘.

[Table 7]

|  | Ex. 1 | Ex. 2 | Ex. 3 | Ex. 4 | Ex. 5 | Ex. 6 |
|---|---|---|---|---|---|---|
| MI (g/10 min.) | 62 | 79 | 98 | 72 | 80 | 65 |
| Tensile strength (MPa) | 57 | 50 | 48 | 52 | 49 | 58 |
| HDT (2) (°C) | 186 | 181 | 183 | 180 | 182 | 188 |
| TVOC (area/g) | 39.9 | 40.8 | 45.6 | 49.7 | 43.2 | 38.8 |
| Fogging test | 2.8 | 2.4 | 2.3 | 2.7 | 2.2 | 4.5 |
| OIT (min.) | 17.6 | 13.8 | 12.6 | 18.9 | 13.1 | > 60 |
| Injection flow mark | × | × | × | × | × | × |

[Table 8]

|  | C. Ex. 1 | C. Ex. 2 | C. Ex. 3 | C. Ex. 4 | C. Ex. 5 | C. Ex. 6 | C. Ex. 7 |
|---|---|---|---|---|---|---|---|
| MI (g/10 min.) | 58 | 70 | 85 | 92 | 94 | 97 | 85 |
| Tensile strength (MPa) | 55 | 45 | 37 | 35 | 31 | 34 | 39 |
| HDT (2) (°C) | 178 | 178 | 164 | 155 | 148 | 154 | 174 |
| TVOC (area/g) | 90.8 | 98.5 | 120.5 | 168.3 | 142.6 | 1142.6 | 11.3 |
| Fogging test | 2.1 | 4.9 | 5.20 | 8.31 | 11.81 | 9.07 | 5.9 |
| OIT (min.) | 1.1 | 7.4 | 0.5 | 3.8 | 5.6 | 4.3 | 8.8 |
| Injection flow mark | × | × | ○ | ○ | ○ | × | × |

As can be seen from Tables 7 and 8, the recycled polyester resin pellets of Examples 1 to 6 all satisfied the desired numerical ranges in terms of such characteristics as melt flow index (mi), tensile strength, and heat deflection temperature and also were excellent in the evaluation of such characteristics as TVOC, fogging, oxidation induction time, and injection flow mark. In particular, the recycled polyester resin pellets of Examples 1 to 6 had properties at an equivalent level or higher as compared with Comparative Example 1 prepared using Resin D, in which commercially available dimethyl terephthalate, rather than recycled dimethyl terephthalate, was used.

**Claims**

1. A recycled polyester resin composition, which comprises a recycled polyester resin comprising a repeat unit derived from a glycol component and a repeat unit derived from recycled dimethyl terephthalate (rDMT); a hindered phenol-based primary antioxidant; and a phosphorus-based or sulfur-based secondary antioxidant,
   wherein in the recycled polyester resin, the crystallized area value is 20% or more when calculated according to the following Equation 1 for the crystallized region and the non-crystallized region obtained by separating the crystal diffraction peaks appearing at 20 values of 0° to 50° by the individual peak fitting method in an X-ray diffraction (XRD) analysis:

Crystallized area value (%) = (area of crystallized region)/(area of crystallized region + area of non-crystallized region) $\times$ 100.  [Equation 1]

2. The recycled polyester resin composition of claim 1, which comprises the primary antioxidant in an amount of 0.001 part by weight to 0.3 part by weight and the secondary antioxidant in an amount of 0.001 part by weight to 0.2 part by weight, relative to 100 parts by weight of the recycled polyester resin.

3. The recycled polyester resin composition of claim 1, which further comprises a wax-based slip agent comprising at least one selected from the group consisting of fatty acid ester, fatty acid amide, polyethylene wax, vegetable wax, and mineral wax.

4. The recycled polyester resin composition of claim 3, wherein the content of the wax-based slip agent is 0.001 part by weight to 0.2 part by weight relative to 100 parts by weight of the recycled polyester resin.

5. The recycled polyester resin composition of claim 1, which further comprises at least one additive selected from the group consisting of flame retardants, nucleating agents, coupling agents, fillers, plasticizers, impact modifiers, lubricants, antibacterial agents, release agents, inorganic additives, ultraviolet stabilizers, antistatic agents, pigments, and dyes.

6. The recycled polyester resin composition of claim 1, wherein the recycled polyester resin composition has a melt flow index (MI) of 60 g/10 minutes or more as measured under the conditions of 250°C and 2.16 kg according to ASTM D1238,

   a tensile strength of 45 MPa or more according to ASTM D638, and
   a heat deflection temperature (HDT) of 175°C or higher as measured at a low load of 0.45 MPa according to ASTM D648.

7. The recycled polyester resin composition of claim 1, wherein the recycled polyester resin composition has a TVOC peak area of 80 area/g or less as measured by a gas chromatography/mass spectrometry (GC/MS) analysis method.

8. The recycled polyester resin composition of claim 1, wherein the recycled polyester resin composition has a change in haze of 5 or less according to the following Equation 2:

$$[\text{Equation 2}]$$

$$\text{Change in haze} = |H1 - H2|$$

   in Equation 2, H1 is the haze measured before fogging evaluation under the conditions of 130°C and 5 hours according to the DIN 75201-A test method for pellets prepared using the recycled polyester resin composition, which is a numerical value excluding the unit, and
   H2 is the haze due to gas formed during fogging evaluation under the conditions of 130°C and 5 hours according to the DIN 75201-A test method for pellets prepared using the recycled polyester resin composition, which is a numerical value excluding the unit.

9. The recycled polyester resin composition of claim 1, wherein, when pellets prepared using the recycled polyester resin composition are dried under a reduced pressure at 50°C for 15 hours and heated from 30°C to 240°C at 10°C/minute using a differential scanning calorimeter (DSC, TA Instruments) in a nitrogen atmosphere, and when the conditions are then changed from nitrogen to air at a conversion rate of 20 ml/minute, the oxidation exotherm time measured while 240°C is maintained for 60 minutes is 10 minutes or more.

10. The recycled polyester resin composition of claim 1, wherein the recycled polyester resin has a negative value of the difference in the crystallized area value according to the following Equation 3:

[Equation 3]

$$\text{Difference in crystallized area value} = Ca_1 - Ca_2$$

in Equation 3, $Ca_1$ is the crystallized area value calculated according to the above Equation 1 for a polyester resin comprising a repeat unit derived from dimethyl terephthalate, which is a numerical value excluding the unit, and $Ca_2$ is the crystallized area value calculated according to the above Equation 1 for the recycled polyester resin comprising a repeat unit derived from recycled dimethyl terephthalate (rDMT), which is a numerical value excluding the unit.

11. The recycled polyester resin composition of claim 10, wherein the difference in the crystallized area value according to the above Equation 3 is -0.5 or less.

12. The recycled polyester resin composition of claim 1, wherein the recycled polyester resin has a total content (% by mole) of an ethylene glycol residue and a diethylene glycol residue of 5% by mole or less based on the total number of moles of the glycol component residues.

13. The recycled polyester resin composition of claim 1, wherein the recycled dimethyl terephthalate has a purity of 90% or more and an APHA color value of 120 or less.

14. The recycled polyester resin composition of claim 1, wherein the recycled dimethyl terephthalate is prepared by depolymerizing waste polyester.

15. The recycled polyester resin composition of claim 1, wherein the recycled dimethyl terephthalate is prepared by first depolymerizing waste polyester to obtain recycled bis(2-hydroxyethyl) terephthalate (rBHET) and second depolymerizing it.

16. The recycled polyester resin composition of claim 1, wherein the recycled polyester resin has a melting point of 210°C or higher and a melting enthalpy ($\Delta$Hm) of 38 J/g or more as measured in a second scan using a differential scanning calorimeter.

17. The recycled polyester resin composition of claim 1, wherein in the recycled polyester resin has an intrinsic viscosity at 35°C of 0.5 dl/g to 1.5 dl/g, a color-b of 12 or less, and a color-L of 70 or more.

18. The recycled polyester resin composition of claim 1, which has a melting point of 210°C or higher and a melting enthalpy ($\Delta$Hm) of 38 J/g or more as measured in a second scan using a differential scanning calorimeter.

19. The recycled polyester resin composition of claim 1, wherein the recycled polyester resin has a heat deflection temperature (HDT) of 90°C or higher as measured at a low load of 0.48 MPa according to ASTM D648.

20. A molded article, which is prepared from the recycled polyester resin composition of claim 1.

21. The molded article of claim 20, wherein the molded article is a molded article for a vehicle headlamp.

[Fig. 1]

[Fig. 2]

EP 4 707 331 A1

[Fig. 3]

[Fig. 4]

[Fig. 5]

[Fig. 6]

## INTERNATIONAL SEARCH REPORT

| International application No. |
| --- |
| **PCT/KR2024/018265** |

| | |
| --- | --- |
| **A. CLASSIFICATION OF SUBJECT MATTER** | |

**C08K 5/00**(2006.01)i; **C08K 5/13**(2006.01)i; **C08K 5/37**(2006.01)i; **C08K 5/49**(2006.01)i; **C08K 5/098**(2006.01)i; **C08K 5/17**(2006.01)i; **C08L 67/02**(2006.01)i; **C08G 63/183**(2006.01)i; **C08J 11/10**(2006.01)i; **C07C 69/82**(2006.01)i

According to International Patent Classification (IPC) or to both national classification and IPC

**B. FIELDS SEARCHED**

Minimum documentation searched (classification system followed by classification symbols)

C08K 5/00(2006.01); B29C 48/08(2019.01); B29C 48/18(2019.01); C07C 51/43(2006.01); C07C 51/487(2006.01); C07C 67/03(2006.01); C07C 69/82(2006.01); C08G 18/66(2006.01); C08G 63/183(2006.01); C08G 63/199(2006.01); D01F 6/70(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 재생 디메틸 테레프탈레이트(regenerated dimethyl terephthalate), 폴리에스테르 수지(polyester resin), 산화 방지제(antioxidant), 해중합(depolymerization)

**C. DOCUMENTS CONSIDERED TO BE RELEVANT**

| Category* | Citation of document, with indication, where appropriate, of the relevant passages | Relevant to claim No. |
| --- | --- | --- |
| Y | CN 115594581 B (ZHEJIANG JIAREN NEW MATERIALS CO., LTD.) 14 July 2023 (2023-07-14) <br> paragraphs [0019]-[0022], [0056] | 1-21 |
| Y | KR 10-2024-0024420 A (SK CHEMICALS CO., LTD.) 26 February 2024 (2024-02-26) <br> paragraphs [0002], [0027], [0072], [0082], [0083]; claims 1, 3 | 1-21 |
| A | CN 116043353 A (ZHENGZHOU ZHONGYUAN SPANDEX ENGINEERING TECHNOLOGY CO., LTD.) 02 May 2023 (2023-05-02) <br> paragraphs [0034], [0039], [0040], [0042]-[0054] | 1-21 |
| A | KR 10-2024-0071303 A (KOLON INDUSTRIES, INC.) 22 May 2024 (2024-05-22) <br> paragraphs [0084], [0086], [0087]; claim 23 | 1-21 |
| A | KR 10-2022-0041177 A (EASTMAN CHEMICAL COMPANY) 31 March 2022 (2022-03-31) <br> paragraphs [0011], [0813]; claim 1 | 1-21 |

☐ Further documents are listed in the continuation of Box C.  ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **03 April 2025** | **03 April 2025** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.

**PCT/KR2024/018265**

| Patent document cited in search report | | | Publication date (day/month/year) | Patent family member(s) | | | Publication date (day/month/year) |
|---|---|---|---|---|---|---|---|
| CN | 115594581 | B | 14 July 2023 | CN | 115594581 | A | 13 January 2023 |
| KR | 10-2024-0024420 | A | 26 February 2024 | WO | 2024-039116 | A1 | 22 February 2024 |
| CN | 116043353 | A | 02 May 2023 | | None | | |
| KR | 10-2024-0071303 | A | 22 May 2024 | WO | 2024-106889 | A1 | 23 May 2024 |
| KR | 10-2022-0041177 | A | 31 March 2022 | CN | 114423803 | A | 29 April 2022 |
| | | | | CN | 114423803 | B | 29 October 2024 |
| | | | | EP | 4004094 | A1 | 01 June 2022 |
| | | | | US | 2022-0325036 | A1 | 13 October 2022 |
| | | | | WO | 2021-021902 | A1 | 04 February 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20110080260

**Non-patent literature cited in the description**

- *CHEMICAL ABSTRACTS*, 6683-19-8
- *CHEMICAL ABSTRACTS*, 23128-74-7
- *CHEMICAL ABSTRACTS*, 29598-76-3
- *CHEMICAL ABSTRACTS*, 31570-04-4
- *CHEMICAL ABSTRACTS*, 25728-82-9